# EUROPEAN PATENT APPLICATION

(11) **EP 1 829 860 A1**
(43) Date of publication of application: **05.09.2007**
(21) Application number: 05811796.1
(22) Date of filing: 05.12.2005
(51) Int. Cl.: C07C 401/00, A61K 31/59, A61P 3/02, A61P 17/06

(54) **9,10-SECOPREGNANE DERIVATIVE AND MEDICINE**

(30) Priority: 03.12.2004 JP 2004351611; 16.09.2005 JP 2005269459
(71) Applicant: Nippon Shinyaku Co., Ltd., Kyoto-shi, Kyoto 601-8550 (JP)
(72) Inventor: FUJIEDA, Hiroki, Kobe-shi Hyogo;6580073 (JP); OTSU, Hironori, 5800015 (JP); YASUFUKU, Shoji, 5200246 (JP)
(74) Representative: Vossius & Partner
(86) International application number: PCT/JP2005/022309
(87) International publication number: WO 2006/059768

(57) **Abstract**

The object of the present invention is to provide a novel and useful vitamin D₃ derivative which does not have much effect on the systemic calcium metabolism while retaining an excellent vitamin D₃ activity.

The present invention may include a 9,10-secopregnane derivative represented by the following general formula [1] and a pharmaceutical composition containing the same as an active ingredient.

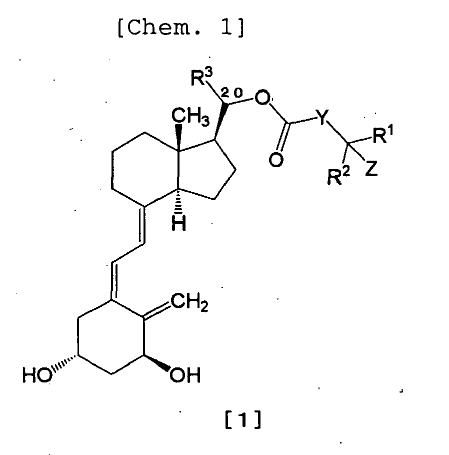

In the general formula [1], Y represents (1) a single bond, (2) alkylene, (3) alkenylene or (4) phenylene; R¹ and R² are same or different and each represents (1) hydrogen, (2) alkyl or (3) cycloalkyl, R¹ and R² taken together with the carbon atom adjacent thereto may form a cycloalkyl; R³ represents hydrogen or methyl; and Z represents (1) hydrogen, (2) hydroxy or (3) -NR¹¹R¹².

## Description

### [Technical Field]

The present invention relates to a 9,10-secopregnane derivative (vitamin D₃ derivative) and a pharmaceutical composition containing the same as an active ingredient.

### [Background Art]

Psoriasis vulgaris, ichthyosis synndrome, keratosis of palm and sole, pustulosis of palm and sole and lichen pilaris are keratotic disorders in a broad sense showing various characteristic skin signs such as erythema, wetting, hypertrophy, keratinization and scale. This disease is an intractable chronic disease and causes a big obstacle to comfortableness of daily life of patients. With regard to its pathological background, it has been believed to be based on disorder of growth and differentiation of both inflammatory cells and skin cells.
Psoriasis vulgaris which is a representative disease among keratotic disorders is not fatal, but it is intractable and is accompanied by prejudice for its appearance and also by mental pain. Therefore, there are many cases where quality of life (QOL) is deteriorated significantly.

Many therapeutic methods have been applied for the above-mentioned keratosis such as psoriasis vulgaris. There is, however, no radical therapy and symptomatic treatment and care over a long period of time have been performed. As a main therapeutic method, external application of adrenocorticosteroidal agents has been widely adopted achieving an excellent therapeutic effect. However, there is also a strong side effect and induction of skin atrophy and rebound has been considered to be a problem in particular.
In recent years, topical application of vitamin D₃ derivatives having a 9,10-secopregnane skeleton has been widely used. As compared with steroids, this topically applicable agent has fewer side effects and has an effect to prolong the term before the recurrence is noted (refer, for example, to Non-Patent Document 1). It has been believed that the vitamin D₃ derivative is effective for keratosis including psoriasis vulgaris via a suppressive action on the growth of epidermal cells (refer, for example, to Non-Patent Documents 2 and 3), a promotional action on epidermal cell differentiation (refer, for example, to Non-Patent Documents 4 to 6), a suppressive action on cytokine production and a suppressive action on the activation of T cells (refer, for example, to Non-Patent Document 7), etc.

With regard to a vitamin D₃ derivative having a 9,10-secopregnane skeleton, there have been known many derivatives such as (1S,3R,20S)-20-(3-hydroxy-3-methylbutyl-oxy)-9,10-secopregna-5Z,7E,10(19)-trien-1,3-diol (generic name: maxacalcitol; hereinafter, this compound will be referred to as maxacalcitol) (its synthetic method and pharmacological actions are mentioned, for example, in Patent Document 1 and its pharmacological actions are mentioned, for example, in Non-Patent Documents 8 to 10) and the compounds mentioned in Patent Document 2 or Non-Patent Document 11.

On the other hand, it has been well known that 1α,25(OH)₂D₃ which is an active form of vitamin D raises levels of serum calcium concentration together with the levels of serum parathyroid hormone whereby calcium homeostasis is maintained. The most anxious side effects of vitamin D₃ derivatives which have been clinically used at present are dry mouth, malaise, torpor, anorexia, vomiting, abdominal pain and muscular weakness as a result of a rise in calcium concentration in serum (hypercalcemia). Accordingly, it is necessary to periodically measure the calcium concentration in blood not only in the case where administration is given to patients suffering from hypercalcemia but also in patients who are not suffering from said disease. There is also a limitation for its dose (refer, for example, to Non-Patent Documents 12 and 13).

Consequently, there has been an earnest desire for a vitamin D₃ derivative which does not have much effect on the systemic calcium metabolism and is able to specifically normalize the keratotic disorders of epidermal cells, as a therapeutic agent for keratosis such as psoriasis vulgaris.

Patent Document 1: EP publication No. 0184112
Patent Document 2: Japanese Patent No. 2908566
Patent Document 3: US Patent No. 6296997
Patent Document 4: US Patent No. 5612325
Non-Patent Document 1: Kobayashi J, et al, Nishinihonhihuka, 60, 882 (1998)
Non-Patent Document 2: Kondo S, et al, Arch Dermatol Res, 292, 550(2000)
Non-Patent Document 3: Kobayashi T, et al, J Eur Acad Dermatol Venereol, 5, 132(1995)
Non-Patent Document 4 : Kragballe K, et al, Arch Dermatol Res, 282, 169 (1990)
Non-Patent Document 5: Matsunaga T, et al, J Dermatol, 17, 135(1990)
Non-Patent Document 6: Takahashi H, et al, J Dermatol Sci, 31, 21 (2003)
Non-Patent Document 7: Komine M, et al, Arch Dermatol Res, 291, 500 (1999)
Non-Patent Document 8: Chem. Pharm. Bull., 39(12), 3221-3224(1991)
Non-Patent Document 9: Chem. Pharm. Bull., 40(6), 1494-1499(1992)
Non-Patent Document 10: Chem. Pharm. Bull., 44(12), 2280-2286 (1996)
Non-Patent Document 11: Steroids, 59, 686(1994)
Non-Patent Document 12: Mizutani J, IYAKU Journal, 39, 122(2003)
Non-Patent Document 13: Nakagawa H, IYAKU Journal, 39, 93(2003)
Non-Patent Document 14: Bull. Chem. Soc. Jpn., 52(7), 1989-1993(1979)
Non-Patent Document 15: Chem. Pharm. Bull., 44(12), 2280 (1996)
Non-Patent Document 16: Bioorg. Med. Chem. Lett., 2, 1713(1992)
Non-Patent Document 17: Tetrahedron Lett., 45, 7837(2004)
Non-Patent Document 18: J. Chem. Soc., 115, 1207(1919)
Non-Patent Document 19: J. of Pharmacology and Experimental Therapeutics, 305, 675(2003)
Non-Patent Document 20: J. Chem. Soc. Perkin Trans.1, 7, 1951(1990)
Non-Patent Document 21: Bull. Chem. Soc. Jpn, 67, 293 (1994)
Non-Patent Document 22: J. Org. Chem., 33, 1839(1968)
Non-Patent Document 23: Chem. Pharm. Bull., 34(10), 4410-4413 (1986)
Non-Patent Document 24: J. Nutr.Sci. Vitaminol., 26, 545-556 (1980)
Non-Patent Document 2 5: J. Org. Chem., 66(23), 7832-7840(2001)
Non-Patent Document 26: Tetrahedron, 42(11), 2931-2935(1986)
Non-Patent Document 27: Tetrahedron Lett., 33,41; 6193-6196(1992)
Non-Patent Document 28: Synthesis, 134-135 (1983)
Non-Patent Document 29: J. Org. Chem., 68(1), 27-34(2003)
Non-Patent Document 30: Yakugaku Zasshi, 72, 1172(1952)
Non-Patent Document 31: J. Med. Chem., 31(2), 428-32(1988)
Non-Patent Document 32: J. Chem. Soc., 115, 1207(1919)
Non-Patent Document 33: J. Am. Chem. Soc., 80, 4969-4971(1958)
Non-Patent Document 34: Tetrahedron, 42(11), 2931-2935(1986)
Non-Patent Document 35: Tetrahedron., 42(11), 2931-2935(1986)
Non-Patent Document 36: Synthesis, 7, 1009-1014(1998)
Non-Patent Document 37: Tetrahedron Lett., 28(15), 1685-1688(1987)
Non-Patent Document 38: J. Chem. Soc., 503-506(1946)
Non-Patent Document 39: Tetrahedron Lett., 2749-2752(1976)
Non-Patent Document 40: Archiv der Phamazie, 316, 339-346(1983)
Non-Patent Document 41: J. Med. Chem., 11, 138-140(1968)
Non-Patent Document 42: J. Med. Chem., 43, 1508-1518(2000)
Non-Patent Document 43: J. Gen. Chem. USSR(Engl.Transl.), 32, 786-788(1962)
Non-Patent Document 44: J. Org. Chem., 52, 4798-4800(1987)

### [Disclosure of the Invention]

### [Problem to be Solved by the Invention]

The main object of the present invention is to provide a novel vitamin D₃ derivative which does not have much effect on the systemic calcium metabolism while retaining excellent vitamin D₃ activity.

### [Means to Solve the Problem]

The present inventors have extensively conducted various investigations and found that a novel 9,10-secopregnane derivative which will be mentioned below or a pharmaceutically acceptable salt thereof achieves the above object and accomplished the present invention.
The present invention may include a 9,10-secopregnane derivative represented by the following general formula. [1] (hereinafter, referred to as the compound of the present invention) or a pharmaceutically acceptable salt thereof. A characteristic feature of the compound of the present invention in terms of the structure is that a carbonyloxy group is directly bound to carbon of position 20 without being mediated by an alkylene chain. 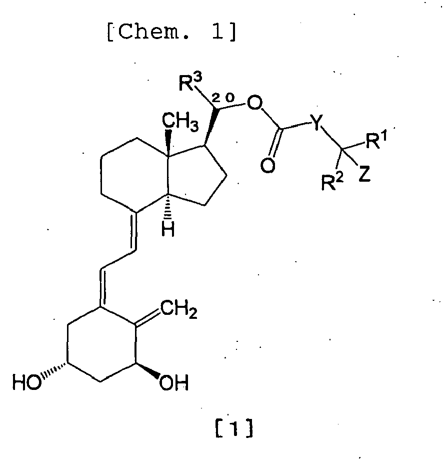 In the general formula [1],
Y represents (1) a single bond, (2) a C₁₋₅ alkylene which may be substituted by one to three member(s) selected from the group consisting of halogen, hydroxy and oxo, (3) a C₁₋₅ alkenylene or (4) phenylene;
R¹ and R² are same or different and each represents (1) hydrogen, (2) a C₁₋₆ alkyl which may be substituted by one to three halogen (s) or (3) a C₃₋₈ cycloalkyl, or R¹ and R² taken together with the carbon atom adjacent thereto may form a C₃₋₈ cycloalkyl;
R³ represents hydrogen or methyl; and
Z represents (1) hydrogen, (2) hydroxy or (3) - NR¹¹R¹² in which R¹¹ represents hydrogen or a C₁₋₆ alkyl and R¹² represents (1) a C₁₋₆ alkyl which may be substituted by hydroxy or (2) a C₁₋₆ alkylsulfonyl.

The present invention may also include a pharmaceutical composition comprising the compound of the present invention or a pharmaceutically acceptable salt thereof as an active ingredient, a therapeutic agent for keratiotic disorders including psoriasis vulgaris comprising the compound of the present invention or a pharmaceutically acceptable salt thereof as an active ingredient, and a process for producing the compound of the present invention or a pharmaceutically acceptable salt thereof.
Examples of the preferable compound of the present invention may include, for example, the following compounds (1)to (33):
(1) (1S,3R,20S)-20-(4-hydroxy-4-methylpentanoyloxy)-9,10-secopregna-5Z,7E,10(19)-trien-1,3-diol,
(2) (1S,3R,20S)-20-(3-hydroxy-3-methylbutanoyloxy)-9,10-secopregna-5Z,7E,10(19)-trien-1,3-diol,
(3) (1S,3R,20S)-20-(5-hydroxy-5-methylhexanoyloxy)-9,10-secopregna-5Z,7E,10(19)-trien-1,3-diol,
(4) (1S,3R,20S)-20-(4,4,4-trifluoro-3-hydroxy-3-methylbutanoyloxy)-9,10-secopregna-52,7E,10(19)-trien-1,3-diol,
(5) (1S,3R,20S)-20-(3-hydroxy-4-methylpentanoyloxy)-9,10-secopregna-5Z,7E,10(19)-trien-1,3-diol,
(6) (1S,3R,20S)-20-(4,4,4-trifluorobutanoyloxy)-9,10-secopregna-5Z,7E,10(19)-trien-1,3-diol,
(7) (1S,3R,20S)-20-[4,4,4=trifluoro-3-hydroxy-3-(trifluoromethyl)butanoyloxy]-9,10-secopregna-5Z,7E,10(19)- trien-1,3-diol,
(8) (1S,3R,20S)-20-[(2E)-4-hydroxy-4-methylpent-2-enoyloxy]-9,10-secopregna-5Z,7E,10(19)-trien-1,3-diol,
(9) (1S,3R,20S)-20-(3-cyclopropyl-3-hydroxypropanoyloxy)-9,10-secopregna-5Z,7E,10(19)-trien-1,3-diol,
(10) (1S,3R,20S)-20-[(2E)-4-ethyl-4-hydroxyhex-2-enoyloxy]-9,10-secopregna-5Z,7E,10(19)-trien-1,3-diol,
(11) (1S,3R,20S)-20-(5-hydroxy-5-methylheptanoyloxy)-9,10-secopregna-5Z,7E,10(19)-trien-1,3-diol,
(12) (1S, 3R, 20S) -20- (3-ethyl-3-hydroxypentanoyloxy) -9, 10-secopregna-5Z, 7E, 10 (19) -trien-1, 3-diol,
(13) (1S, 3R, 20S) -20- (4-ethyl-4-hydroxyhexanoyloxy) -9, 10-secopregna-5Z, 7E, 10 (19) - trien - 1,3 - diol,
(14) (1S,3R,20S)-20-[3-(1-hydroxy-1-methylethyl)-benzoyloxy]-9,10-secopregna-5Z,7E,10(19)-trien-1,3-diol,
(15) (1S,3R,20S)-20-[N-(isopropylsulfonyl)-3-aminopropanoyloxy]-9,10-secopregna-5Z,7E,10(19)-trien-1,3-diol,
(16) (1S,3R,205)-20-(6-hydroxy-6-methylheptanoyloxy)-9,10-secopregna-5Z,7E,10(19)-trien-1,3-diol,
(17) (1S,3R,20S)-20-{4-[2,2,2-trifluoro-1-hydroxy-1-(trifluoromethyl)ethyl]benzoyloxy}-9,10-secopregna-5Z,7E,10(19)-trien-1,3-diol,
(18) (1S,3R,20S)-20-(5,5,5-trifluoropentanoyloxy)-9,10-secopregna-5Z,7E,10(19)-trien-1,3-diol,
(19) (1S,3R,20S)-20-[N-(2-hydroxy-2-methylpropyl)-N-methyl-2-aminoacetyloxy]-9,10-secopregna-5Z,7E,10(19)-trien-1,3-diol,
(20) (1S,3R,20S)-20-[3-(1-hydroxycyclopentyl)-propanoyloxy]-9,10-secopregna-5Z,7E,10(19)-trien-1,3-diol,
(21) (S,3R,20S)-20-(3,3-difluoro-4-hydroxy-4-methylpentanoyloxy)-9,10-secopregna-5Z,7E,10(19)-trien-1,3-diol,
(22) (1S,3R,20S)-20-[(3S)-3,4-dihydroxy-4-methylpentanoyloxy] -9, 10-secopregna-5Z, 7E, 10 (19) -trien-1,3-diol,
(23) (1S, 3R, 20S) -20- (5, 5, 5-trifluoro-4-hydroxy-4-methylpentanoyloxy)-9,10-secopregna-5Z,7E,10(19)-trien-1,3-diol,
(24) (1S,3R,20R)-20-(4-hydroxy-4-methylpentanoyloxy)-9,10-secopregna-5Z,7E,10(19)-trien-1,3-diol,
(25) (1S, 3R, 20R) -20- (3-hydroxy-3-methylbutanoyloxy) -9, 10-secopregna-5Z,7E,10(19)-trien-1,3-diol,
(26) (1S, 3R, 17β)-17-(4-hydroxy-4-methylpentanoyloxymethyl)-9,10-secoandrosta-5Z,7E,10(19)-trien-1,3-diol,
(27) (1S,3R,20S)-20-(4-hydroxy-4-methyl-3-oxopentanoyloxy)-9,10-secopregna-5Z,7E,10(19)-trien-1,3-diol,
(28) (1S, 3R, 20S) -20- (4-ethyl-4-hydroxy-3-oxohexanoyloxy)-9,10-secopregna-5Z, 7E, 10(19)-trien-1,3-diol,
(29) (1S,3R,20S)-20-[3-(hydroxymethyl)phenylacetyloxy]-9, 10-secopregna-5Z, 7E, 10 (19)-trien-1,3-diol,
(30) (1S,3R,17β)-17-[(2E)-4-ethyl-4-hydroxyhex-2-enoyloxymethyl]-9,10-secoandrosta-5Z,7E,10(19)-trien-1,3-diol,
(31) (1S, 3R, 20S)-20- [(3R)-3,4-dihydroxy-4-methylpentanoyloxy]-9,10-secopregna-5Z,7E,10(19)-trien-1,3-diol,
(32) (1S, 3R, 20S) -20- [5, 5, 5-trifluoro-4-hydroxy-4-(trifluoromethyl)pentanoyloxy]-9,10-secopregna-5Z,7E,10(19)-trien-1,3-diol, and
(33) (1S, 3R, 20S) -20- (3-hydroxy-3-n-propylhexanoyloxy) - 9,10-secopregna-5Z,7E,10(19)-trien-1,3-diol.
The present invention will now be described in detail.
In the present invention, examples of the "halogen" may include fluorine, chlorine, bromine and iodine.
In the present invention, examples of the "alkylene" may include straight or branched alkylene containing 1 to 5 carbons such as methylene, ethylene, trimethylene, methylethylene, tetramethylene, methyltrimethylene, ethylethylene, pentamethylene, methyltetramethylene and ethyltrimethylene. Particularly, the alkylene having 1 to 3 carbon atoms are preferred. The alkylene of the present invention may be substituted by one to three members selected from the group consisting of halogen, hydroxy and oxo.
In the present invention, examples of the "alkenylene" may include straight or branched alkenylene containing 2 to 5 carbons such as ethenylene, propenylene, butenylene and pentenylene. Particularly, the alkenylene having 2 to 4 carbon atoms are preferred.
In the present invention, examples of the "phenylene" may include 1,2-phenylene, 1,3-phenylene and 1,4-phenylene.
In the present invention, examples of the "alkyl" may include straight or branched alkyl containing 1 to 6 carbons such as methyl, ethyl, n-propyl, isopropyl, n-butyl, isobutyl, sec-butyl, tert-butyl, n-pentyl, isopentyl, n-hexyl and isohexyl. Particularly, methyl and ethyl are preferred. The alkyl of the present invention may be substituted by one to three halogen(s).
The alkyl moiety of "alkylsulfonyl" and "trialkylsilyl" may include the above-mentioned alkyl.
In the present invention, examples of the "cycloalkyl" may include C₃₋₈ cycloalkyl having one to three ring(s) such as cyclopropyl, cyclobutyl, cyclopentyl, cyclohexyl, cycloheptyl, cyclooctyl, cyclononyl, cyclodecanyl, adamantyl (e.g., 1-adamantyl, 2-adamantyl), 2-bicyclo[3.1.1]heptyl and 2-bicyclo[2.2.1]heptyl. Particularly, C₃₋₆ cycloalkyl having one to three ring(s) are preferred.
In the present invention, examples of the "acyl" may include alkanoyl having 1 to 6 carbons such as formyl, acetyl, propionyl, butyryl and isobutyryl. Particularly, the acyl having 2 to 5 carbon atoms are preferred.
In the present invention, the protective groups are not particularly limited insofar as they are protective groups for hydroxyl which are able to be used in the present reaction and may include 1) trialkylsilyl such as triethylsilyl, tributylsilyl and tert-butyl dimethylsilyl, 2) (2-trimethylsilyl)ethoxymethyl, 3) aromatic methyl such as benzyl and 4-methoxyphenylmethyl, 4) acyl such as acetyl and 5) 2-tetrahydropyranyl.

### [Brief Description of the Drawing]

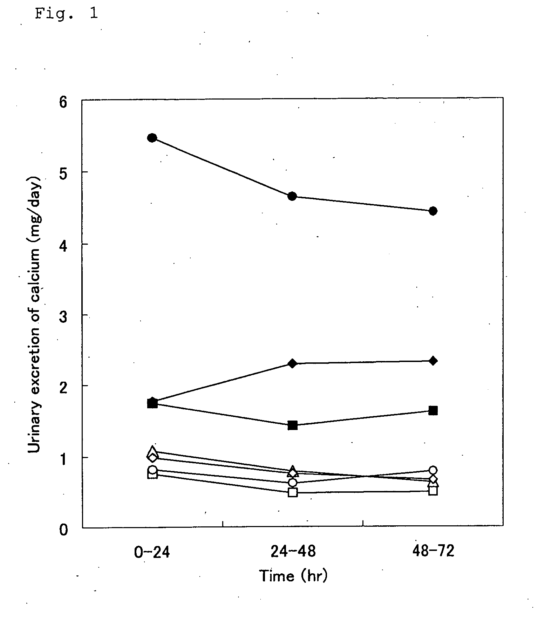

[Fig. 1] It shows the result of repetitive percutaneous administration of the compound of the present invention mentioned in Example 1 and maxacalcitol for three days once daily to male SD strain of rats (7 weeks of age). The vertical axis shows the amount of urinary excretion of calcium (mg/day) while the horizontal axis shows time (hour(s)). Open triangles show a control; open squares show the case where 0.33 µg/kg of the compound of Example 1 was administered; open rhombuses show the case where 3.3 µg/kg of the compound of Example 1 was administered; open circles show the case where 33 µg/kg of the compound of Example 1 was administered; black squares show the case where 0.33 µg/kg of maxacalcitol was administered; black rhombuses show the case where 3.3 µg/kg of maxacalcitol was administered; and black circles show the case where 33 µg/kg of maxacalcitol was administered.

### [Best Mode for Carrying Out the Invention]

The compound of the present invention can be produced from known compounds or easily synthesizable intermediates by, for example, the following process. When raw materials have a substituent which affects the reaction in the production of the compound of the present invention, it is usual that the raw materials are subjected to the reaction after being previously protected with an appropriate protective group by a known method. The protective group can be removed by a known method after the reaction is completed.

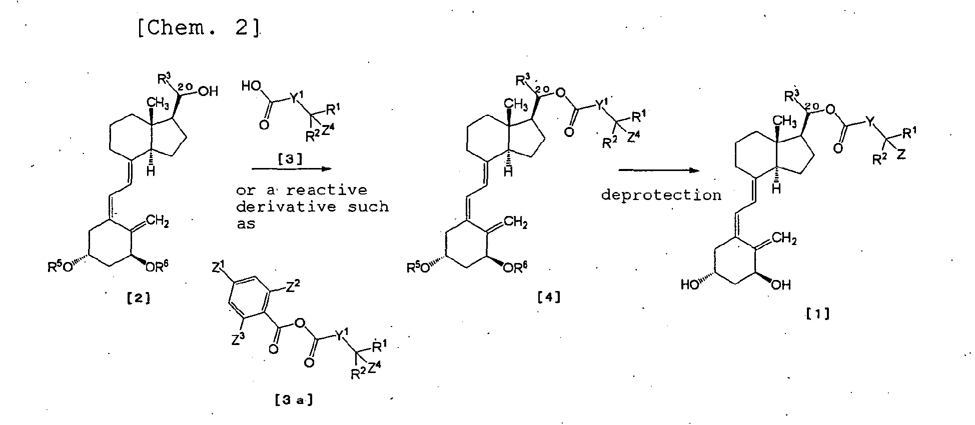

[Wherein Y, Z, R¹, R² and R³ are the same as defined above. Y¹ represents (1) a single bond, (2) a C₁₋₅ alkylene which may be substituted by 1 to 3 member(s) selected from the group consisting of halogen, protected hydroxy and oxo, (3) a C₁₋₅ alkenylene or (4) phenylene. R⁵ and R⁶ represent protective groups for hydroxy. Z¹, Z² and Z³ are same or different and each represents halogen. Z⁴ represents (1) hydrogen, (2) protected hydroxy or (3) -NR¹³R¹⁴. R¹³ represents hydrogen or a C₁₋₆ alkyl and R¹⁴ represents (1) a C₁₋₆ alkyl which may be substituted by a protected hydroxy or (2) a C₁₋₆ alkylsulfonyl.]
This reaction is a condensation reaction of a compound (alcohol) represented by the general formula [2] with a compound (carboxylic acid) represented by the general formula [3] followed by a deprotecting reaction and, therefore, is carried out by the methods known by themselves as a condensation reaction and a deprotecting reaction. For example, the above alcohol is made to react with the above carboxylic acid and then a deprotecting reaction is carried out whereby the compound of the present invention is able to be produced.

### First step (condensation reaction)

This is a step where condensation of the alcohol [2] with the carboxylic acid [3] is carried out at the reaction temperature of -20°C to 100°C in the presence or absence of a base (for example, an organic base including triethylamine, N,N-diisopropyl-N-ethylamine, N,N-dimethylaniline, pyridine, 4-dimethylaminopyridine and 1,8-diazabicyclo[5.4.0]undec-7-ene) using a condensing agent (for example, 1,1'-oxalyldiimidazole, 1-ethyl-3-(3-dimethylaminoproyl)carbodiimide, dicyclohexylcarbodiimide, diethyl cyanophosphonate, diphenyl phosphoryl azide and 2-chloro-1-methylpyridinium iodide). The solvent which can be used is not particularly limited insofar as it does not participate in the reaction and its examples may include ethers such as tetrahydrofuran and diethyl ether, amides such as N,N-dimethylformamide and N,N-dimethylacetamide, nitriles such as acetonitrile and propionitrile, hydrocarbons such as benzene and toluene, halogenated hydrocarbons such as chloroform and dichloromethane and a mixed solvent thereof. In the present step, it is also possible to add an additive (for example, 1-hydroxybenzotriazole and N-hydroxysuccinimide).
Although the reaction time varies depending upon the type of material and condensing agent, the reaction temperature, etc., it is usually appropriate to be from 30 minutes to 24 hours. The amounts of the above carboxylic acid [3] and condensing agent used are preferred to be one to three mole(s) to one mole of the alcohol [2].
Instead of the above carboxylic acid [3] used in the present step, it is also possible to use a reactive derivative thereof. Examples of the reactive derivative are those which are commonly used for an ester condensation forming reaction such as acid halide (for example, acid chloride and acid bromide), mixed anhydride, imidazolide and active amide. When the reaction of the present step is carried out using said reactive derivative, the above condensing agent may not be used.
When a mixed anhydride, for example, is used as a reactive derivative of the carboxylic acid [3], the condensation reaction can be carried out at the reaction temperature of -20°C to 100°C using a pyridine solvent such as pyridine and 4-methylpyridine or using the same base and solvent as those mentioned above. It is also possible to add, as an additive, 4-dimethylaminopyridine for example. The reaction time varies depending upon the type of the mixed anhydride used and the reaction temperature and, usually, it is appropriate to be from 30 minutes to 24 hours. The particularly preferred mixed anhydride in the present step where mixed anhydride is used may include mixed anhydride represented by the following general formula [3a] (refer, for example, to Non-Patent Document 14) . 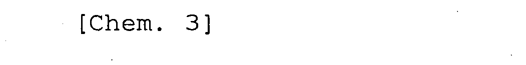 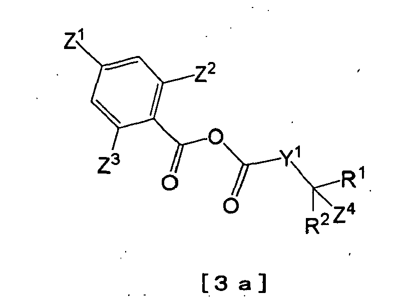 [Wherein Y¹, R¹, R², Z¹, Z², Z³ and Z⁴ are the same as defined above.]
R⁵ and R⁶ are not particularly limited insofar as they are protective groups for hydroxyl which can be used in the present reaction and they may include the protective groups as mentioned above.

The compound represented by the general formula [2] which is a raw material compound in which R³ is methyl can be produced by the same process as mentioned, for example, in Non-Patent Documents 15 and 16.
The compound represented by the general formula [2] which is a raw material compound in which R³ is hydrogen can be produced by the same process as mentioned, for example, in Non-Patent Document 17.

The compound represented by the general formula [3] which is a raw material compound can be produced by the same process as mentioned, for example, in Non-Patent Documents 18 to 22 or by a process similar to the process mentioned in Patent Document 4' and Non-Patent Documents 26 to 41.

### Second step (deprotecting reaction)

This step is a deprotecting reaction of hydroxy and can be carried out by a conventional method per se. To be more specific, although being different depending upon the type of the protective group used, the deprotection may be carried out as follows when, for example, a tertbutyldimethylsilyl group is used as a protective group.
For a deprotecting reaction of the compound represented by the general formula [4], a deprotective agent (for example, tetrabutylammonium fluoride, hydrogen fluoride, hydrogen fluoride-pyridine, acetic acid and trifluoroacetic acid) may be used and the reaction may be carried out at the reaction temperature of -20°C to 100°C. The solvent which can be used is not particularly limited insofar as it does not participate in the reaction and its examples may include ethers such as tetrahydrofuran and diethyl ether, amides such as N,N-dimethylformamide and N,N-dimethylacetamide, nitriles such as acetonitrile and propionitrile, hydrocarbons such as benzene and toluene, halogenated hydrocarbons such as chloroform and dichloromethane and a mixed solvent thereof. Although the reaction time varies depending upon the type of the raw material and the deprotecting agent, the reaction temperature, etc., it is usually appropriate to be from 30 minutes to 24 hours. The amount of the deprotecting agent is preferred to be 1 to 100 mole(s) to one mole of the compound represented by the general formula [4].

The compound of the present invention may include a compound having asymmetric carbon and the present invention covers not only an optically active substance thereof but also a racemic compound thereof. Synthesis of such an optically active substance can be carried out by means of resolution using a chiral column according to the conventional method, while it is also possible to produce it by means of an asymmetric synthesis of the compound [2] which is a raw material compound (for example, it can be produced by the same manner as in an asymmetric synthesis mentioned in Non-Patent Documents 15 and 16).
When there is a geometric isomer or a tautomeric isomer in the compound of the present invention, the compound of the present invention covers not only any of such isomers but also a mixture thereof.

The compound of the present invention may be used as a medicament in a form of a free base, it is also possible to use it by converting it into a form of a pharmaceutically acceptable salt by a known method. Examples of such a salt may include salts of mineral acids such as hydrochloric acid, hydrobromic acid, sulfuric acid and phosphoric acid and salts of organic acids such as acetic acid, citric acid, tartaric acid, maleic acid, succinic acid, fumaric acid, p-toluenesulfonic acid, benzenesulfonic acid and methanesulfonic acid.
For example, a hydrochloride of the compound of the present invention can be produced by dissolving the compound of the present invention in a solution of hydrogen chloride in alcohol, ethyl acetate or ether.

As shown in the test examples below, the compound of the present invention is useful as a medicament and is particularly useful as a therapeutic agent for keratosis disorders such as psoriasis vulgaris.
When the compound of the present invention is administered as a medicine, the compound of the present invention can be administered to mammals including human either as it is or as a pharmaceutical composition containing, the compound of the present invention at the concentration of, for example, 0.0001% to 99.5% or, preferably, 0.001% to 90% in a pharmaceutically acceptable non-toxic and inert carrier.
With regard to the carrier, one or more auxiliary agent (s) for pharmaceutical formulations such as a solid, semi-solid or liquid diluent, a filler and other auxiliary agents for pharmaceutical formulations may be used. It is desirable that the pharmaceutical composition is administered as a unit dose form. Although there is no particular limitation for the unit dose form of the pharmaceutical composition concerning the present invention, it goes without saying that administration is performed by a dose form which is suitable for the administering method. Topical administration (for example, percutaneous administration) is preferred.
It is desirable that the dose as a treating agent for keratiotic disorders including psoriasis vulgaris is set by taking account of state of a patient such as nature and degree of the disease, age and body weight and administering method and, usually, the dose to an adult is generally within a range of 0.01 mg/human to 1,000 mg/human or, preferably, 0.1 mg/human to 500 mg/human as the amount of the active ingredient of the compound of the present invention.
In some cases, the dose less than the above may be sufficient and, in some other cases, the dose more than the above may be necessary. It is also possible to administer by dividing the dose into two to five times a day.

### [Examples]

The present invention will now be described' in more detail by way of Reference Examples, Examples, Test Examples and Formulation Examples of the compound of the present invention, to which, however, the present invention is not limited.

### Reference Example 1: 3-(tert-Butyldimethylsilyloxy)-3-methylbutyric acid

Step 1: 4-Dimethylaminopyridine (0.78 g) was added to a solution of 3.76 g of 3-hydroxy-3-methylbutyric acid and 4.13 g of benzyl alcohol in anhydrous methylene chloride and the mixture was stirred under cooling with ice. After 9.9 g of N,N'-dicyclohexylcarbodiimide was added thereto, the ice bath was removed and the mixture was stirred overnight. After the insoluble deposit was filtered off, the mother liquor was concentrated to give 13 g of the residual oily product. This product was purified by a silica gel column chromatography to give 7.2 g of benzyl 3-hydroxy-3-methylbutyrate as a light yellow oily product.
   ¹H-NMR (CDCl₃) δ: 1.28 (6H, s), 2. 55 (2H, s), 5.16 (2H, s), 7.36(5H, s)
Step 2: 2,6-Lutidine (3.6 g) was added to a solution of 3.5 g of benzyl 3-hydroxy-3-methylbutyrate prepared in the step 1 in anhydrous methylene chloride and the mixture was stirred under cooling with ice. Tert-Butyl dimethylsilyltrifluoromethanesulfonate (3.9 mL) was slowly dropped thereinto and the mixture was stirred under cooling with ice for 1 hour and then at room temperature for 2 hours. The reaction solution was diluted with ethyl acetate, washed with water, a saturated aqueous solution of ammonium chloride and a saturated aqueous solution of sodium chloride, dried over anhydrous magnesium sulfate and concentrated. The residue (4.5 g) was purified by a silica gel column chromatography to give 2.62 g of benzyl 3-(tert-butyldimethylsilyloxy)-3-methylbutyrate as, a colorless oily product.
   ¹H-NMR (CDCl₃) δ: 0.07(6H, s), 0.82 (9H, s), 1.36(6H, s), 2.52(2H, s), 5.09 (2H, s), 7.35(5H, s)
Step 3: Benzyl 3-(tert-butyldimethylsilyloxy)-3-methylbutyrate (2.37 g) prepared in the step 2 was dissolved in 30 mL of ethyl acetate, then 0.47 g of 10% palladium carbon was added thereto and the mixture was hydrogenated with stirring at ordinary pressure. After 40 minutes, stirring was stopped and the catalyst was filtered off. The solvent of the filtrate was evaporated *in vacuo* to give 1.70 g of the objective compound as a colorless oily product.
   ¹H-NMR (CDCl₃) δ: 0.18 (6H, s), 0.89(9H, s), 1.40(6H, s), 2.51 (2H, s).

### Reference Example 2: (1S,3R,20S)-1,3-Bis(tert-butyldimethylsilyloxy)-9,10-secopregna-5Z;7E,10(19)-trien-20-ol

A solution of 155 mg of (1S,3R,20S)-1,3-bis(tert-butyldimethylsilyloxy)-pregna-5,7-dien-20-ol (its synthetic method is mentioned, for example, in Non-Patent Document 23) in 500 mL of tetrahydrofuran was bubbled with argon gas under cooling with ice for 10 minutes. After that, a 500-watt high pressure mercury lamp whose cooling layer was circulated with a filter solution of nickel sulfate-copper sulfate (it is mentioned, for example, in Non-Patent Document 24) was inserted into a reaction bath and the reaction solution was irradiated for 5 minutes with stirring under cooling with ice. After a further irradiation of 2.5 minutes, the reaction solution was transferred to a brown flask and heated to reflux for 3 hours. After the solvent was evaporated *in vacuo,* the residue was purified by a silica gel column chromatography and a preparatory thin layer chromatography to give 30 mg of the objective,compound as a colorless oily product.
¹H-NMR (CDCl₃) δ: 0.06 (12H, s), 0.54(3H, s), 0.88(18H, s), 1.23(3H,d),2.45(1H, dd),2.84(1H,dd),3.71(2H,m), 4.19(1H, m),4.38(1H, dd),4.86(1H, d),5.18(1H, s), 6.03(1H, d),6.23(1H,d)

### Example, 1: (1S,3R,20S)-20-(4-Hydroxy-4-methyl-pentanoyloxy)-9,10-secopregna-5Z,7E,10(19)-trien-1,3-diol

Step 1: 2,4,6-Trichlorobenzoyl chloride (13 µL) was added to a solution of 20 mg of 4-triethylsilyloxy-4-methylpentanoic acid produced in accordance with the method mentioned in Non-Patent Document 25 and 11.2 µL of triethylamine in 0.5 mL of tetrahydrofuran, and the mixture was stirred for 30 minutes at room temperature. After the crystals separated therefrom were filtered off, tetrahydrofuran was evaporated therefrom and the residue was dried *in vacuo.* To the residue were added a solution of 30 mg of (1S, 3R, 20S)-1,3-bis (tert-butyldimethylsilyloxy)-9,10-secopregna-5Z,7E,10(19)-trien-20-ol prepared in Reference Example 2 in 0.5 mL of anhydrous benzene and 30 mg of 4-dimethylaminopyridine in an argon atmosphere and the mixture was stirred for 30 minutes at room temperature. After the reaction solution was diluted with ethyl acetate, it was washed with a saturated aqueous solution of sodium hydrogen carbonate and a saturated saline solution in this order, dried over anhydrous magnesium sulfate and concentrated *in vacuo.* The residue was purified by a silica gel column chromatography to give 27 mg of (1S,3R,20S)-20-(4-triethylsilyloxy-4-methylpentanoyloxy)-1,3-bis(tert-butyldimethylsilyloxy)-9,10-secopregna-5Z,7E,10(19)-triene as a colorless oily product.
   ¹H-NMR (CDCl₃) δ: 0.06(12H, s), 0. 54 (3H, s), 0.57 (6H, q), 0.87(18H, s), 0.94(9H, t), 1.20(6H, s), 2.36(2H, t), 2.84(1H, d), 4.19(1H, m), 4.37(1H, dd), 4.85(1H, d), 4.94(1H, m), 5.17(1H, d), 6.02(1H, d), 6.23(1H, d)
Step 2: A reagent prepared by an addition of 16 µL of acetic acid to 0.56 mL of 1M tetra(n-butyl) ammonium fluoride was added, in an argon atmosphere, to a solution of 22 mg of (1S,3R,20S)-20-(4-triethylsilyloxy-4-methylpentanoyloxy)-1,3-bis(tert-butyldimethylsilyloxy)-9,10-secopregna-5Z,7E,10(19)-triene prepared in the step 1 in 1 mL of anhydrous tetrahydrofuran and the mixture was stirred overnight at room temperature. Cold water and ethyl acetate were added to the reaction solution to perform the extraction procedure and then the ethyl acetate layer was washed with a saturated saline solution, dried over anhydrous magnesium sulfate and concentrated *in vacuo.* The residue was purified by a silica gel column chromatography and a preparative thin layer chromatography to give 7.1 mg of the objective compound of the present invention as colorless powder.
   ¹H-NMR (CDCl₃) δ: 0.55(3H, s), 1.23(6H, s), 1.23(3H, d), 1.80(2H, t), 2.39(2H, t), 2.60(1H, dd), 2.83(1H, m), 4.24 (1H, m), 4.43 (1H, dd), 4.95 (1H, m), 4.99 (1H, dd), 5.33 (1H, br), 6.02 (1H, d), 6.37 (1H, d)
   (+)-FAB MS m/z 447 [M + H]⁺

### Example 2: (1S,3R,20S)-20-(3-Hydroxy-3-methylbutanoyloxy)-9,10-secopregna-5Z,7E,10(19)-trien-1,3-diol

Step 1: (1S,3R,20S)-20-[3-(tert-butyldimethylsilyloxy)-3-methylbutanoyloxy]-1,3-bis(tert-butyldimethylsilyloxy)-9,10-secopregna-5Z,7E,10(19)-triene was produced by the same process as in the step 1 of Example 1 using 3-(tert-butyldimethylsilyloxy)-3-methylbutanoic acid (Reference Example 1) instead of 4-triethylsilyloxy-4-methylpentanoic acid.
   ¹H-NMR (CDCl₃) δ: 0.06(12H, s), 0.08(6H, s), 0.53(3H, s), 0.84(9H, s), 0.87(18H, s), 1.23(3H, d), 1.40(3H, s), 1.36(3H, s), 2.43(2H, brs), 2.84(1H, d), 4.18(1H, m), 4.36(1H, dd), 4.85(1H, d), 4.91(1H, m), 5.17(1H, d), 6.02(1H,d), 6.23(1H,d)
Step 2: The objective compound of the present invention was produced by the same process as in the step 2 of Example 1 using (1S, 3R, 20S) -20- [3- (tert-butyldimethylsilyloxy)-3-methylbutanoyloxy]-1,3-bis(tert-butyldimethylsilyloxy)-9,10-secopregna-5Z,7E,10(19)-triene produced in the above step 1 instead of (1S,3R,20S)-20-(4-triethylsilyloxy-4-methylpentanoyloxy)-1,3-bis(tert-butyldimethylsilyloxy)-9,10-secopregna-5Z,7E,10(19)-triene.
   ¹H-NMR(CDCl₃) δ: 0.56(3H, s), 1.26(3H, d), 1.27(6H, s), 2.32(1H, dd), 2.44(2H, s), 2.60(1H, dd), 2.84(1H, m), 3.74(1H, br), 4.24(1H, m), 4.43(1H, dd), 4.99(1H, s), 5.01(1H, m), 5.33(1H, s), 6.03(1H, d), 6.37(1H, d)
   ESI MS m/z 455 [M + Na]⁺

### Example 3: (1S,3R,20S)-20-(5-Hydroxy-5-methylhexanoyloxy)-9,10-secopregna-5Z,7E,10(19)-trien-1,3-diol

Step 1: (1S,3R,20S)-20-(5-triethylsilyloxy-5-methylhexanoyloxy)-1,3-bis(tert-butyldimethylsilyloxy)-9,10-secopregna-5Z,7E,10(19)-triene was produced by the same process as in the step 1 of Example 1 using 5-(triethylsilyloxy-5-methyhexanoic acid produced by the process mentioned in Non-Patent Document 26 instead of 4-triethylsilyloxy-4-methylpentanoic acid.
Step 2: (1S,3R,20S)-20-(5-triethylsilyloxy-5-methylhexanoyloxy)-1,3-bis(tert-butyldimethylsilyloxy)-9,10-secopregna-5Z,7E,10(19)-triene produced in the above step 1 was subjected to the same reaction as in the step 2 of Example 1 to produce the objective compound of the present invention.
   (+)-ESI MS m/z 483.5 [M + Na]⁺

### Example 4: (1S,3R,20S)-20-(4,4,4-Trifluoro-3-hydroxy-3-methylbutanoyloxy)-9,10-secopregna-5Z,7E,10(19)-trien-1,3-diol

Step 1: (1S,3R,20S)-20-[3-(tert-butyldimethylsilyloxy)-4,4,4-trifluoro-3-methylbutanoyloxy]-1,3-bis(tert-butyldimethylsilyloxy)-9,10-secopregna-5Z,7E,10(19)-triene was produced by the same process as in the step 1 of Example 1 using 3-(tert-butyldimethylsilyloxy)-4,4,4-trifluoro-3-methylbutanoic acid instead of 4-triethylsilyloxy-4-methylpentanoic acid.
Step 2: (1S,3R,20S)-20-[3-(tert-butyldimethylsilyloxy)-4,4,4-trifluoro-3-methylbutanoyloxy]-1,3-bis(tert-butyldimethylsilyloxy)-9,10-secopregna-5Z,7E,10(19)-triene produced in the above step 1 was subjected to the same reaction as in the step 2 of Example 1 to produce the objective compound of the present invention.
   (+)-ESI MS m/z 509.3 [M + Na]⁺

### Example 5: (1S,3R,20S)-20-(3-Hydroxy-4-methylpentanoyloxy)-9,10-secopregna-5Z,7E,10(19)-trien-1,3-diol

Step 1: (1S,3R,20S)-20-[3-(tert-butyldimethylsilyloxy)-4-methylpentanoyloxy]-1,3-bis(tert-butyldimethylsilyloxy)-9,10-secopregna-5Z,7E,10(19)-triene was produced by the same process as in the step 1 of Example 1 using 3-(tert-butyldimethylsilyloxy)-4-methylpentanoic acid produced by the process mentioned in Non-Patent Document 27 instead of 4-triethylsilyloxy-4-methylpentanoic acid.
Step 2: (1S,3R,20S)- 20-[3-(tert-butyldimethylsilyloxy)-4-methylpentanoyloxy]-1,3-bis(tert-butyldimethylsilyloxy)-9,10-secopregna-5Z,7E,10(19)-triene produced in the above step 1 was subjected to the same reaction as in the step 2 of Example 1 to produce the objective compound of the present invention.
   (+)-ESI MS m/z 469.4 [M + Na]⁺

### Example 6: (1S,3R,20S)-20-(4,4,4-Trifluorobutanoyloxy)-9,10-secopregna-5Z,7E,10(19)-trien-1,3-diol

Step 1: (1S,3R,20S)-20-(4,4,4-trifluorobutanoyloxy)-1,3-bis(tert-butyldimethylsilyloxy)-9,10-secopregna-5Z,7E,10(19)-triene was produced by the same process as in the step 1 of Example 1 using 4,4,4-trifluoroacetic acid instead of 4-triethylsilyloxy-4-methylpentanoic acid.
Step 2: (1S,3R,20S)-20-(4,4,4-trifluorobutanoyloxy)-1,3-bis(tert-butyldimethylsilyloxy)-9,10-secopregna-5Z,7E,10(19)-triene produced in the above step 1 was subjected to the same reaction as in the step 2 of Example 1 to produce the objective compound of the present invention.
   (+)-ESI MS m/z 479.3 [M + Na]⁺

### Example 7: (1S,3R,20S)-20-[4,4,4-Trifluoro-3-hydroxy-3-(trifluoromethyl)butanoyloxy]-9,10-secopregna-5Z,7E,10(19)-trien-1,3-diol

Step 1: (1S,3R,20S)-20-[3-(tert-butyldimethylsilyloxy)-4,4,4-trifluoro-3-(trifluoromethyl)butanoyloxy]-1,3-bis(tert-butyldimethylsilyloxy)-9,10-secopregna-5Z,7E,10(19)-triene was produced by the same process as in the step 1 of Example 1 using 3-(tert-butyldimethylsilyloxy)-4,4,4-trifluoro-3-(trifluoromethyl)butanoic acid instead of 4-triethylsilyloxy-4-methylpentanoic acid.
Step 2: (1S,3R,20S)-20-[3-(tert-butyldimethylsilyloxy)-4,4,4-trifluoro-3-(trifluoromethyl)butanoyloxy]-1,3-bis(tert-butyldimethylsilyloxy)-9,10-secopregna-5Z,7E,10(19)-triene produced in the above step 1 was subjected to the same reaction as in the step 2 of Example 1 to produce the objective compound of the present invention.
   (-) -FAB MS m/z 539.2 [M - H]⁻

### Example 8: (1S,3R,20S)-20-[(2E)-4-Hydroxy-4-methylpent-2-enoyloxy]-9,10-secopregna-5Z,7E,10(19)-trien-1,3-diol

Step 1: (15,3R,20S)-20-[(2E)-4-triethylsilyloxy-4-methylpent-2-eoyloxy]-1,3-bis(tert-butyldimethylsilyloxy)-9,10-secopregna-5Z,7E,10(19)-triene was produced by the same process as in the step 1 of Example 1 using (2E) -4-triethylsiloxy-4-methylpent-2-enoic acid produced by the process mentioned in Non-Patent Document 28 instead of 4-triethylsilyloxy-4-methylpentanoic acid.
Step 2: (1S,3R,20S)-20-[(2E)-4-triethylsilyloxy-4- , methylpent-2-eoyloxy]-1,3-bis(tert-butyldimethylsilyloxy)-9,10-secopregna-5Z,7E,10(19)-triene produced in the above step 1 was subjected to the same reaction as in the step 2 of Example 1 to produce the objective compound of the present invention.
   (+)-ESI MS m/z 467.3 [M + Na]⁺

### Example 9: (1S,3R,20S)-20-(3-Cyclopropyl-3-hydroxy-propanoyloxy)-9,10-secopregna-5Z,7E,10(19)-trien-1,3-diol

Step 1: (1S, 3R, 20S)-20-[3-(tert-butyldimethylsilyloxy)-3-cyclopropylpropanoyloxy]-1,3-bis(tert-butyldimethylsilyloxy)-9,10-secopregna-5Z,7E,10(19)-triene was produced by the same process as in the step 1 of Example 1 using 3-(tert-butyldimethylsilyloxy)-3-cyclopropylpropioic acid produced by the process mentioned in Non-Patent Document 29 instead of 4-triethylsilyloxy-4-methylpentanoic acid.
Step 2: (1S,3R,20S)-20-[3-(tert-butyldimethylsilyloxy)-3-cyclopropylpropanoyloxy]-1,3-bis(tert-butyldimethylsilyloxy)-9,10-secopregna-5Z,7E,10(19)-triene produced in the above step 1 was subjected to the same reaction as in the step 2 of Example 1 to produce the objective compound of the present invention,
   (+)-ESI MS m/z 467.3 [M + Na]⁺

### Example 10: (1S,3R,20S)-20-[(2E)-4-Ethyl-4-hydroxyhex-2-enoyloxy]-9,10-secopregna-5Z,7E,10(19)-trien-1,3-diol

Step 1: (1S,3R,20S)-20-[(2E)-4-(tert-butyldimethylsilyloxy)-4-ethylhex-2-enoyloxy]-1,3-bis(tert-butyldimethylsilyloxy)-9,10-secopregna-5Z,7E,10(19)-triene was produced by the same process as in the step 1 of Example 1 using (2E)-4-(tert-butyldimethylsilyloxy)-4-ethylhex-2-enoic acid produced by the process mentioned in Non-Patent Document 28 instead of 4-triethylsilyloxy-4-methylpentanoic acid.
Step 2: (1S,3R,20S)-20-[(2E)-4-(tert-butyldimethylsilyloxy)-4-ethylhex-2-enoyloxy]-1,3-bis(tert-butyldimethylsilyloxy)-9,10-secopregna-5Z,7E,10(19)-triene produced in the above step 1 was subjected to the same reaction as in the step 2 of Example 1 to produce the objective compound of the present invention.
   (+)-ESI MS m/z 495.5 [M + Na]⁺

### Example 11: (1S,3R,20S)-20-(5-Hydroxy-5-methylheptanoyloxy)-9,10-secopregna-5Z,7E,10(19)-trien-1,3-diol

Step 1: (1S,3R,20S)-20-(5-triethylsilyloxy-5-methylheptanoyloxy) -1, 3-bis (tert-butyldimethylsilyloxy)-9,10-secopregna-5Z,7E,10(19)-triene was produced by the same process as in the step 1 of Example 1 using 5-triethylsilyloxy-5-methylheptanoic acid produced by the process mentioned in Non-Patent Document 30 instead of 4-triethylsilyloxy-4-methylpentanoic acid.
Step 2: (1S,3R,20S)-20-(5-triethylsilyloxy-5-methylheptanoyloxy)-1,3-bis(tert-butyldimethylsilyloxy)-9,10-secopregna-5Z,7E,10(19)-triene produced in the above step 1 was subjected to the same reaction as in the step 2 of Example 1 to produce the objective compound of the present invention.
   (+)-ESI MS m/z 475.3 [M + Na]⁺

### Example 12: (1S,3R,20S)-20-(3-Ethyl-4-hydroxypentanoyloxy)-9,10-secopregna-5Z,7E,10(19)-trien-1,3-diol

Step 1: (1S, 3R, 20S)-20-[3-ethyl-3-triethylsilyloxypentanoyloxy]-1,3-bis(tert-butyldimethylsilyloxy)-9,10-secopregna-5Z,7E,10(19)-triene was produced by the same process as in the step 1 of Example 1 using 3-ethyl-3-triethylsilyloxypentanoic acid produced by the process mentioned in Non-Patent Document 31 instead of 4-triethylsilyloxy-4-methylpentanoic acid.
Step 2: (1S,3R,20S)-20-[3-ethyl-3-triethylsilyloxypentanoyloxy]-1,3-bis (tert-butyldimethylsilyloxy)-9,10-secopregna-5Z,7E,10(19)-triene produced in the above step 1 was subjected to the same reaction as in the step 2 of Example 1 to produce the objective compound of the present invention.
   (+)-ESI MS m/z 483.4 [M + Na]⁺

### Example 13: (1S,3R,20S)-20-(4-Ethyl-4-hydroxyhexanoyloxy)-9,10-secopregna-5Z,7E,10(19)-trien-1,3-diol

Step 1: (1S,3R,20S)-20-(4-ethyl triethylsilyloxyhexanoyloxy)-1,3-bis(tert-butyldimethylsilyloxy)-9,10-secopregna-5Z,7E,10(19)-triene was produced by the same process as in the step 1 of Example 1 using 4-ethyl-4-triethylsilyloxyhexanoic acid produced by the process mentioned in Non-Patent Document 32 instead of 4-triethylsilyloxy-4-methylpentanoic acid.
Step 2: (1S,3R,20S)-20-(4-ethyl-4-triethylsilyloxyhexanoyloxy)-1,3-bis(tert-butyldimethylsilyloxy)-9,10-secopregna-5Z,7E,10(19)-triene produced in the above step 1 was subjected to the same reaction as in the step 2 of Example 1 to produce the objective compound of the present invention.
   (+)-ESI MS m/z 497.5 [M + Na]⁺

### Example 14: (1S,3R,20S)-20-[3-(1-Hydroxy-1-methylethyl)-benzoyloxy]-9,10-secopregna-5Z,7E,10(19)-trien-1,3-diol

Step 1: (1S,3R,20S)-20-[3-(1-triethylsilyloxy-1-methylethyl)benzoyloxy]-1,3-bis(tert-butyldimethylsilyloxy)-9,10-secopregna-5Z,7E,10(19)-triene was produced by the same process as in the step 1 of Example 1 using 3-(1-triethylsilyloxy-1-methylethyl)benzoic acid produced by the process mentioned in Non-Patent Document 33 instead of 4-triethylsilyloxy-4-methylpentanoic acid.
Step 2: (1S,3R,20S)-20-[3-(1-triethylsilyloxy-1-methylethyl)benzoyloxy]-1,3-bis(tert-butyldimethylsilyloxy)-9,10-secopregna-5Z,7E,10(19)-triene produced in the above step 1 was subjected to the same reaction as in the step 2 of Example 1 to produce the objective compound of the present invention.
   (+)-ESI MS m/z 517.4 [M + Na]⁺

### Example 15: (1S,3R,20S)-20-[N-(Isopropylsulfonyl)-3-aminopropanoyloxy]-9;10-secopregna-5Z,7E,10(19)-trien-1,3-diol

Step 1: (1S,3R,20S)-20-[N-(isopropylsulfonyl)-3-aminopropanoyloxy]-1,3-bis(tert-butyldimethylsilyloxy)-9,10-secopregna-5Z,7E,10(19)-triene was produced by the same process as in the step 1 of Example 1 using N-(isopropylsulfonyl)-β-alanine produced by the process mentioned in Patent Document 3 instead of 4-triethylsilyloxy-4-methylpentanoic acid.
Step 2: (1S,3R,20S)-20-[N-(isopropylsulfonyl)-3-aminopropanoyloxy]-1,3-bis(tert-butyldimethylsilyloxy)-9,10-secopregna-5Z,7E,10(19)-triene produced in the above step 1 was subjected to the same reaction as in the step 2 of Example 1 to produce the objective compound of the present invention.
   (+)-ESI MS m/z 532.4 [M + Na]⁺

### Example 16: (1S, 3R, 20S) -20- (6-Hydroxy-6-methylheptanoyloxy)-9,10-secopregna-5Z,7E,10(19)-trien-1,3-diol

Step 1: (1S,3R,20S)-20-(-6-triethylsilyloxy-6-methylheptanoyloxy)-1,3-bis(tert-butyldimethylsilyloxy)-9,10-secopregna-5Z,7E,10(19)-triene was produced by the same process as in the step 1 of Example 1 using 6-triethylsilyloxy-6-methylheptanoic acid produced by the process mentioned in Non-Patent Document 35 instead of 4-triethylsilyloxy-4-methylpentanoic acid.
Step 2: (1S,3R,20S)-20-(6-triethylsilyloxy-6-methylheptanoyloxy)-1,3-bis(tert-butyldimethylsilyloxy)-9,10-secopregna-5Z,7E,10(19)-triene produced in the above step 1 was subjected to the same reaction as in the step 2 of Example 1 to produce the objective compound of the present invention.
   (+)-ESI MS m/z 497.4 [M + Na]⁺

### Example 17: (1S,3R,20S)-20-{4-[2,2,2-Trifluoro-1-hydroxy-1-(triflubromethyl)ethyl]benzoyloxy}-9,10-secopregna-5Z,7E,10(19)-trien-1,3-diol

Step 1 : (1S,3R,20S)-20-[4-{2,2,2-trifluoro-1-trifluoromethyl-1-[2-(trimethylsilyl)ethoxymethyloxy]ethyl}benzoyloxy]-1,3-bis(tert-butyldimethylsilyloxy)-9,10-secopregna-5Z,7E,10(19)-triene was produced by the same process as in the step 1 of Example 1 using 4-{2,2,2-trifluoro-1-trifluoromethyl-1-[2-(trimethyksilyl)ethoxymethyloxy]ethyl}benzoic acid instead of 4-triethylsilyloxy-4-methylpentanoic acid.
Step 2: (1S,3R,20S)-20-[4-{2,2,2-trifluoro-1-trifluoromethyl-1- [2-(trimethylsilyl)ethoxymethyloxy]ethyl}benzoyloxy]-1,3-bis(tert-butyldimethylsilyloxy)-9,10-secopregna-5Z,7E,10(19)-triene produced in the above step 1 was subjected to the same reaction as in the step 2 of Example 1 to produce the objective compound of the present invention.

### Example 18: (1S,3R,20S)-20-(5,5,5-Trifluoropentanoyloxy)-9,10-secopregna-5Z, 7E, 10 (19)-trien-1,3-diol

Step 1: (1S,3R,20S)-20-(5,5,5-trifluoropentanoyloxy)-1,3-bis(tert-butyldimethylsilyloxy)-9,10-secopregna-5Z,7E,10(19)-triene was produced by the same process as in the step 1 of Example 1 using 5,5,5-trifluoropentanoic acid instead of 4-triethylsilyloxy-4-methylpentanoic acid.
Step 2: (1S,3R,20S)-20-(5,5,5-trifluoropentanoyloxy)-1,3-bis(tert-butyldimethylsilyloxy)-9,10-secopregna-5Z,7E,10(19)-triene produced in the above step 1 was subjected to the same reaction as in the step 2 of Example 1 to produce the objective compound of the present invention.
   (+)-ESI MS m/z 493.3 [M + Na]⁺

### Example 19: (1S,3R,20S)-20-[N-(2-Hydroxy-2-methylpropyl)-N-methyl-2-aminoacetyloxy]-9,10-secopregna-5Z,7E,10(19)-trien-1, 3-diol

Step 1: (1S,3R,20S)-20-[N-(2-triethylsilyloxy-2-methylpropyl)-N-methyl-2-aminoacetyloxy]-1,3-bis(tert-butyldimethylsilyloxy)-9,10-secopregna-5Z,7E,10(19)-triene was produced by the same process as in the step 1 of Example 1 using N-(2-triethylsilyloxy-2-methylpropyl)-N-methyl-2-aminoacetic acid produced by the process mentioned in Non-Patent Document 40 instead of 4-triethylsilyloxy-4-methylpentanoic acid.
Step 2: (1S,3R,20S)-20-[N-(2-triethylsilyloxy-2-methylpropyl)-N-methyl-2-aminoacetyloxy]-1,3-bis(tert-butyldimethylsilyloxy)-9,10-secopregna-5Z,7E,10(19)-triene produced in the above step 1 was subjected to the same reaction as in the step 2 of Example 1 to produce the objective compound of the present invention.
   (+)-ESI MS m/z 476.4 [M + 1]⁺

### Example 20: (1S,3R,20S)-20-[3-(1-Hydroxycyclopentyl)propanoyloxy]-9,10-secopregna-5Z,7E,10(19)-trien-1;3-diol

Step 1 : (1S,3R,20S)-20- [3-(1-triethylsilyloxycyclopentyl)propanoyloxy]-1,3-bis(tert-butyldimethylsilyloxy)-9,10-secopregna-5Z,7E,10(19)-triene was produced by the same process as in the step 1 of Example 1 using 3-(1-triethylsilyloxycyclopentyl)propioic acid produced by the process mentioned in Non-Patent Document 36 instead of 4-triethylsilyloxy-4-methylpentanoic acid.
Step 2: (1S,3R,20S)-20-[3-(1-triethylsilyloxycyclopentyl)propanoyloxy]-1,3-bis(tert-butyldimethylsilyloxy)-9,10-secopregna-5Z,7E,10(19)-triene produced in the above step 1 was subjected to the same reaction as in the step 2 of Example 1 to produce the objective compound of the present invention.
   (+)-ESI MS m/z 473.5 [M + 1]⁺

### Example 21: (1S,3R,20S)-20-(3,3-Difluoro-4-hydroxy-4-methylpentanoyloxy)-9,10-secopregna-5Z,7E,10(19)-trien-1,3-diol

Step 1 : (1S, 3R, 20S) -20- (4-triethylsilyloxy-3, 3-difluoro-4-methylpentanoyloxy-1, 3-bis (tert-butyldimethylsilyloxy)-9,10-secopregna-5Z,7E,10(19)-triene was produced by the same process as in the step 1 of Example 1 using 4-triethylsilyloxy-3,3-difluoro-4-methylpentanoic acid produced by the process mentioned in Patent Document 4 instead of 4-triethylsilyloxy-4-methylpentanoic acid.
Step 2: (1S, 3R, 20S)-20-(4-triethylsilyloxy-3,3-difluoro-4-methylpentanoyloxy-1,3-bis(tert-butyldimethylsilyloxy)-9,10-secopregna-5Z,7E,10(19)-triene produced in the above step 1 was subjected to the same reaction as in the step 2 of Example 1 to produce the objective compound of the present invention.
   (+)-ESI MS m/z 483.4 [M + 1]⁺

### Example 22: (1S,3R,20S)-20-[(3S)-3,4-Dihydroxy-4-methylpentanoyloxy)-9,10-secopregna-5Z,7E,10(19)-trien-1,3-diol

Step 1: (1S, 3R, 20S)-20-[(3S)-3, 4-bis(triethylsilyloxy)-4-methylpentanoyloxy]-1,3-bis(tert-butyldimethylsilyloxy) -9, 10-secopregna-5Z, 7E, 10 (19) -triene was produced by the same process as in the step 1 of Example 1 using (3S)-3,4-bis(triethylsilyloxy)-4-methylpentanoic acid produced by the process mentioned in Non-Patent Document 37 instead of 4-triethylsilyloxy-4-methylpentanoic acid.
Step 2: (1S,3R,20S)-20-[(3S)-3,4-bis(triethylsilyloxy)-4-methylpentanoyloxy]-1,3-bis(tert-butyldimethylsilyloxy)-9,10-secopregna-5Z,7E,10(19)-triene produced in the above step 1 was subjected to the same reaction as in the step 2 of Example 1 to produce the objective compound of the present invention.
   (+)-ESI MS m/z 485.4 [M + Na]⁺

### Example 23: (1S,3R,20S)-20-(5,5,5-Trifluoro-4-hydroxy-4-methylpentanoyloxy)-9,10-secopregna-5Z,7E,10(19)-trien-1,3-diol

Step 1 : (1S,3R,20S)-20-[4-(tert-butyldimethylsilyloxy)-5,5,5-trifluoro-4-methylpentanoyloxy]-1,3-bis(tert-butyldimethylsilyloxy)-9,10-secopregna-5Z, 7E, 10 (19)-triene was produced by the same process as in the step 1 of Example 1 using 4-(tert-butyldimethylsilyloxy)-5,5,5-trifluoro-4-methylpentanoic acid produced by the process mentioned in Non-Patent Document 41 instead of 4-triethylsilyloxy-4-methylpentanoic acid.
Step 2: (1S,3R,20S)-20-[4-(tert-butyldimethylsilyloxy)-5,5,5-trifluoro-4-methylpentanoyloxy]-1,3-bis(tert-butyldimethylsilyloxy)-9,10-secopregna-5Z,7E,10(19)-triene produced in the above step 1 was subjected to the same reaction as in the step 2 of Example 1 to produce the objective compound of the present invention.
   (+)-ESI MS m/z 523.3 [M + Na]⁺

### Example 24: (1S, 3R, 20R) -20- (4-Hydroxy-4-methylpentanoyloxy)-9,10-secopregna-5Z,7E,10(19)-trien-1,3-diol

Step 1: (1S,3R,20R)-20-(4-triethylsilyloxy)-4-methylperitanoyloxy)-1,3-bis(tert-butyldimethylsilyloxy)-9,10-secopregna-5Z,7E,10(19)-triene was produced by the same process as in the step 1 of Example 1 using (1S, 3R, 20R)-1,3-bis(tert-butyldimethylsilyloxy)-9,10-secopregna-5Z,7E,10(19)-triene-20-ol produced by the process mentioned in Non-Patent Document 16 instead of (1S,3R,20S)-1,3-bis(tert-butyldimethylsilyloxy)-9,10-secopregna-5Z,7E,10(19)-triene-20-ol.
Step 2: (1S,3R,20R)-20-(4-triethylsilyloxy)-4-methylpentanoyloxy)-1,3-bis(tert-butyldimethylsilyloxy)-9,10-secopregna-5Z,7E,10(19)-triene produced in the above step 1 was subjected to the same reaction as in the step 2 of Example 1 to produce the objective compound of the present invention.
   (+)-ESI MS m/z 469.4 [M + Na]⁺

### Example 25: (1S,3R,20R)-20-(3-Hydroxy-3-methylbutanoyloxy)-9,10-secopregna-5Z,7E,10(19)-trien-1,3-diol

Step 1: (1S,3R,20R)-20-(3-tert-butyldimethylsilyloxy-3-methylbutanoyloxy)-1,3-bis(tert-butyldimethylsilyloxy)-9,10-secopregna-5Z,7E,10(19)-triene was produced by the same process as in the step 1 of Example 2 using (1S, 3R, 20R) -1, 3-bis (tert-butyldimethylsilyloxy)-9,10-secopregna-5Z,7E,10(19)-triene-20-ol produced by the process mentioned in Non-Patent Document 16 instead of (1S,3R,20S)-1,3-bis(tert-butyldimethylsilyloxy)-9,10-secopregna-5Z,7E,10(19)-triene-20-ol.
Step 2: (1S, 3R, 20R) -20- (3-tert-butyldimethylsilyloxy-3-methylbutanoyloxy)-1,3-bis(tert-butyldimethylsilyloxy)-9,10-secopregna-5Z,7E,10(19)-triene produced in the above step 1 was subjected to the same reaction as in the step 2 of Example 2 to produce the objective compound of the present invention.
   (+) - FAB MS m/z 432 [M + 1]⁺

### Example 26: (1S, 3R,17β)-17-(4-Hydroxy-4-methylpentanoyloxymethyl)-9,10-secoandrosta-5Z,7E,10(19)-trien-1,3-diol

Step 1: (1S, 3R, 17β)-17-(4-triethylsilyloxy-4-methylpentanoyloxymethyl)-1,3-bis(tert-butyldimethylsilyloxy)-9,10-secoandrosta-5Z,7E,10(19)-triene was produced by the same process as in the step 1 of Example 1 using (1S,3R,17β)-1,3-bis(tert-butyldimethylsilyloxy)-17-hydroxymethyl-9,10-secoandrosta-5Z,7E,10(19)-triene produced by the process mentioned in Non-Patent Document 17 instead of (1S,3R,20S)-1,3-bis(tert-butyldimethylsilyloxy)-9,10-secopregna-5Z,7E,10(19)-triene-20-ol.
Step 2: (1S, 3R, 17β) -17- (4-triethylsilyloxy-4-methylpentanoyloxymethyl)-1,3-bis(tert-butyldimethylsilyloxy)-9,10-secoandrosta-5Z,7E,10(19)-triene produced in the above step 1 was subjected to the same reaction as in the step 2 of Example 1 to produce the objective compound of the present invention.
   (+)-ESI MS m/z 455.3 [M + Na]⁺

### Example 27: (1S,3R,20S)-20-(4-Hydroxy-4-methyl-3-oxo-pentanoyloxy)-9,10-secopregna-5Z,7E,10(19)-trien-1,3-diol

Step 1: (1S,3R,20S)-20-[4-(tert-butyldimethylsilyloxy)-4-methylpenta-2-ynoyloxy]-1,3-bis(tert-butyldimethylsilyloxy)-9,10-secopregna-5Z,7E,10(19)-triene was produced by the same process as in the step 1 of Example 1 using 4-(tert-butyldimethylsilyloxy)-4-methylpenta-2-ynoic acid produced by the process mentioned in Non-Patent Document 38 instead of 4-triethylsilyloxy-4-methylpentanoic acid.
Step 2: A reagent prepared by an addition of 14 µL of acetic acid to 0.81 mL of 1M tetra-n-butylammonium fluoride was added, in an argon atmosphere, to a solution of 32mg of (1S,3R,20S)-20-[4-(tert-butyldimethylsilyloxy)-4-methylpenta-2-ynoyloxy]-1,3-bis(tert-butyldimethylsilyloxy)-9,10-secopregna-5Z,7E,10(19)-triene prepared in the step 1 in 0.4 mL of anhydrous tetrahydrofuran and the mixture was stirred overnight at room temperature. Water and chloroform were added to the reaction solution to perform the extraction for three times and then the chloroform layer was washed with a saturated saline solution, dried over anhydrous magnesium sulfate and concentrated *in vacuo.* The residue was purified by a silica gel column chromatography to give 9.5 mg of the objective compound of the present invention as a light brown amorphous.
   (+)-ESI MS m/z 483.3 [M + Na]⁺

### Example 28: (1S,3R,20S)-20-(4-Ethyl-4-hydroxy-3-oxo-hexanoyloxy)-9,10-secopregna-5Z,7E,10(19)-trien-1,3-diol

Step 1 : (1S,3R,20S)-20-[4-(tert-butyldimethylsilyloxy)-4-ethylhexa-2-ynoyloxy]-1,3-bis(tert-butyldimethylsilyloxy)-9,10-secopregna-5Z,7E,10(19)-triene was produced by the same process as in the step 1 of Example 1 using 4-triethylsilyloxy-4-ethylhexa-2-ynoic acid produced by the process mentioned in Non-Patent Document 38 instead of 4-triethylsilyloxy-4-methylpentanoic acid.
Step 2: (1S,3R,20S)-20-[4-(tert-butyldimethylsilyloxy)-4-ethylhexa-2-ynoyloxy]-1,3-bis(tert-butyldimethylsilyloxy)-9,10-secopregna-5Z,7E,10(19)-triene produced in the above step 1 was subjected to the same reaction as in the step 2 of Example 27 to produce the objective compound of the present invention.
   (+)-ESI MS m/z 511.5 [M + Na]⁺

### Example 29: (1S,3R,20S)-20-[3-(Hydroxymethyl)phenylacetyloxy]-9,10-secopregna-5Z,7E,10(19)-trien-1,3-diol

Step 1: (1S,3R,20S)-20-[3-(tert-butyldimethylsilyloxymethyl)phenylacetyloxy]-1,3-bis(tert-butyldimethylsilyloxy)-9,10-secopregna-5Z,7E,10(19)-triene was produced by the same process as in the step 1 of Example 1 using 3-(tert-butyldimethylsilyloxymethyl)phenylacetic acid produced by the process mentioned in Non-Patent Document 42 instead of 4-triethylsilyloxy-4-methylpentanoic acid.
Step 2: (1S, 3R, 20S)-20-[3-(tert-butyldimethylsilyloxymethyl)phenylacetyloxy]-1,3-bis(tert-butyldimethylsilyloxy)-9,10-secopregna-5Z,7E,10(19)-triene produced in the above step 1 was subjected to the same reaction as in the step 2 of Example 1 to produce the objective compound of the present invention.
   (+)-ESI MS m/z 503.3 [M + Na]⁺

### Example 30: (1S, 3R, 17β) -17- [(2E)-4-Ethyl-4-hydroxy-hex-2-enoyloxymethyl]-9,10-secoandrosta-5Z,7E,10(19)-trien-1,3-diol

Step 1 : (1S,3R,17β)-17-[(2E)-4-(tert-butyldimethylsilyloxy)-4-ethylhex-2-enoyloxymethyl]-1,3-bis (tert-butyldimethylsilyloxy) -9, 10-secoandrosta-5Z,7E,10(19)-triene was produced by the same process as in the step 1 of Example 10 using (1S,3R;17β)-1,3-bis(tert-butyldimethylsilyloxy)-17-hydroxymethyl-9,10-secoandrosta-5Z,7E,10(19)-triene produced by the process mentioned in Non-Patent Document 17 instead of (1S,3R,20S)-1,3-bis(tert-butyldimethylsilyloxy)-9,10-secopregna-5Z,7E,10(19)-triene-20-ol.
Step 2: (1S,3R,17β)-17-[(2E)-4-(tert-butyldimethylsilyloxy)-4-ethylhex-2-enoyloxymethyl]-1,3-bis(tert-butyldimethylsilyloxy)-9,10-secoandrosta-5Z,7E,10(19)-triene produced in the above step 1 was subjected to the same reaction as in the step 2 of Example 1 to produce the objective compound of the present invention.
   (+) -ESI MS m/z 481.4 [M + Na]⁺

### Example 31: (1S,3R,20S)-20-[(3R)-3,4-Dihydroxy-4-methylpentanoyloxy]-9,10-secopregna-5Z,7E,10(19)-trien-1,3-diol

Step 1: (1S,3R,20S)-20-[(3R)-3,4-bis (triethylsilyloxy)-4-methylpentanoyloxy]-1,3-bis(tert-butyldimethylsilyloxy)-9,10-secopregna-5Z,7E,10(19)-triene was produced by the same process as in the step 1 of Example 1 using (3R)-3,4-bis(triethylsilyloxy)-4-methylpentanoic acid produced by the process mentioned in Non-Patent Document 37 instead of 4-triethylsilyloxy-4-methylpentanoic acid.
Step 2: . (1S, 3R, 20S)-20-[(3R)-3,4-bis(triethylsilyloxy)-4-methylpentanoyloxy]-1,3-bis(tert-butyldimethylsilyloxy)-9,10-secopregna-5Z,7E,10(19)-triene produced in the above step 1 was subjected to the same reaction as in the step 2 of Example 1 to produce the objective compound of the present invention.
   (+)-ESI MS m/z 485.4 [M + Na]⁺

### Example 32: (1S,3R,20S)-20-[5,5,5-Trifluoro-4-hydroxy-4-(trifluoromethyl)pentanoyloxy]-9,10-secopregna-5Z,7E,10(19)-trien-1,3-diol

Step 1: (1S, 3R, 20S)-20-[4-(tert-butyldimethylsilyloxy)-5,5,5-trifluoro-4-(trifluoromethyl)pentanoyloxy]-1,3-bis (tert-butyldimethylsilyloxy)-9,10-secopregna-5Z,7E,10(19)-triene was produced by the same process as in the step 1 of Example 1 using 4-(tert-butyldimethylsilyloxy)-5,5,5-trifluoro-4-(trifluoromethyl)pentanoic acid produced by the process mentioned in Non-Patent Document 41 instead of 4-triethylsilyloxy-4-methylpentanoic acid.
Step 2: (1S, 3R, 20S) -20- [4- (tert-butyldimethylsilyloxy)-5,5,5-trifluoro-4-(trifluoromethyl)pentanoyloxy]-1,3-bis(tert-butyldimethylsilyloxy)-9,10-secopregna-5Z,7E,10(19)-triene produced in the above step 1 was subjected to the same reaction as in the step 2 of Example 1 to produce the objective compound of the present invention.
   (+)-ESI MS m/z 577.3 [M + Na]⁺

### Example 33: (1S,3R,20S.)-20-(3-Hydroxy-3-n-propylhexanoylox.y)-9,10-secopregna-5Z;7E,10(19)-trien-1,3-diol

Step 1: (1S,3R,20S)-20-(3=hydroxy-3-n-propylhexanoyloxy)-1,3-bis(tert-butyldimethylsilyloxy)-9,10-secopregna-5Z,7E,10(19)-triene was produced by the same process as in the step 1 of Example 1 using 3-hydroxy-3-n-propylhexanoic acid produced by the process mentioned in Non-Patent Document 44 instead of 4-triethylsilyloxy-4-methylpentanoic acid.
Step 2: (1S,3R,20S)-20-(3-hydroxy-3-n-propylhexanoyloxy)-1,3-bis(tert-butyldimethylsilyloxy)-9,10-secopregna-5Z,7E,10 (19)-triene produced in the above step 1 was subjected to the same reaction as in the step 2 of Example 1 to produce the objective compound of the present invention.
   (+)-ESI MS m/z 511.5 [M + Na]⁺

### Test Example 1: Investigation of an ability to induce differentiation of a human acute myelogenous leukemia cell line HL-60

It has been known that human acute myelogenous leukemia cell line HL-60 is differentiated into a neutrophil-like cell by active form of vitamin D. Thus, the cells after the differentiation begin to express a surface marker such as CD11 and CD32 and, at the same time, they produce oxygen radical by stimulation with a phorbol ester. In this test, the above-mentioned property was utilized to assess an ability to induce the differentiation of HL-60 cell of the compounds of the present invention mentioned in Examples and maxacalcitol using as an indicator the amount of oxygen radical produced by stimulation with a phorbol ester whereby a vitamin D₃ activity was compared and investigated. When commercially available reagents and kits were used, they were used according to the indications attached thereto.
(Procedure) HL-60 cells (supplied from ATCC) were seeded on a 96-well plate at 5 × 10³ cells/100 µL/well and a test compound in two-fold dilution series from 10⁻⁷ M was added to the second to the twelfth rows (maximum concentration: the second row). The first row was used for blank. Maxacalcitol was added to lines A and B and the compound of the present invention was added to lines C and D. After incubation for 72 hours under a moisturized condition in the presence of 5% CO₂ at 37°C using a CO₂ incubator, 10µL of PBS containing 50 mM WST-1 (manufactured by Takara Bio) and 10 µM phorbol 12,13-didecanoate (manufactured by Sigma) were added to each well followed by incubating for 4 hours more. Absorbance (reference wavelength: 655 nM) at 450 nM of a WST-1 formazan dye generated by conversion from WST-1 by oxygen radical produced from the differentiated HL-60 cells was measured by a Benchmark microplate reader (manufactured by BioRad). An absorbance meter control software MPM 3 (manufactured by BioRad) was used and, from a concentration-absorbance curve, the 50%-maximal effect concentration (hereinafter, referred to as "EC₅₀") of each was determined by a logistic curve regression. The result is shown in Table 1.

**Table 1**

| Tested Compound | EC₅₀ (nM) |
|---|---|
| Maxacalcitol | 5.4 |
| Compound of Example 1 | 2.0 |
| Compound of Example 3 | 2.6 |
| Compound of Example 4 | 0.21 |
| Compound of Example 5 | 1.1 |
| Compound of Example 7 | 0.44 |
| Compound of Example 8 | . 3.1 , |
| Compound of Example 10 | 1.2 |
| Compound of Example 11 | 4.3 |
| Compound of Example 12 | 4.2 |
| Compound of Example 13 | 2.3 |
| Compound of Example 19 | 4.0 |
| Compound of Example 20 | 1.7 |
| Compound of Example 27 | 3.0 |
| Compound of Example 28 | 2.5 |

As shown in Table 1, the compounds of the present invention showed the similar or even stronger ability to induce the cell differentiation as compared with maxacalcitol and it is apparent that they exhibit an excellent vitamin D₃ activity.

### Test Example 2: Investigation on the action to raise calcium concentration in serum

A solution of the compound of the present invention mentioned in Example 1 or maxacalcitol in ethanol containing 0.3% of DMSO was repeatedly subjected to a percutaneous administration (33 µg/200 µL/kg/day) for seven days once daily onto the back of male rats (7 weeks of age) of the SD strain. An ethanol solution containing 0.3% of DMSO was administered to a control group. At 24 hours after the final administration, blood was collected and calcium concentration in serum was measured. The result is shown in Table 2.

**Table 2**

| Tested Compound | Calcium Concentration in Serum (mg/dL) |
|---|---|
| Control | 11.5 |
| Compound of Example 1 | 11.8 |
| Maxacalcitol | 15.0 |

As shown in Table 2, maxacalcitol induced a rise in calcium concentration in serum while the compound of the present invention hardly affected the calcium concentration in serum.

### Test Example 3: Investigation on the action to raise the urinary excretion of calcium (1)

A solution of the compound of the present invention mentioned in Example 1 or maxacalcitol in ethanol containing 0.3% of DMSO was repeatedly subjected to a percutaneous administration (0.33, 3.3 and 33 µg/200 µL/kg/day) for three days once daily onto the back of male rats (7 weeks of age) of the SD strain. An ethanol solution containing 0.3% of DMSO was administered to a control group. Calcium concentration in urine collected every 24 hours after administration on each day was measured and multiplied by the amount of urine to calculate the urinary excretion of calcium. The result is shown in Fig. 1.
As shown in Fig. 1, urinary excretion of calcium increased in a dose-dependent manner in a group to which maxacalcitol was administered while, in the group to which the compound of the present invention was administered, there was almost no difference from the control group whereby no significant increase in urinary excretion of calcium was noted .

### Test Example 4: Investigation on the action to raise the urinary excretion of calcium (2)

The compound of the present invention mentioned in Examples 3 or 8, or maxacalcitol (22.4 nmol/kg) was subjected to an intravenous administration to male rats (7 weeks of age) of the SD strain. A physiological saline containing 0.1% of Triton X 100 was administered to a control group. Calcium concentration in urine collected during 24 hours after the administration was measured and urinary excretion of calcium ([calcium concentration in urine] x [amount of urine]) and ratio of urinary excretion of calcium based on the control group ([calcium amount in urine upon administration of the compound to be tested]/[calcium amount in urine in the control group]) were calculated. The result is shown in Table 3.

**Table 3**

| Tested Compound | Dose (µg/kg) | Urinary excretion of calcium (mg) | Ratio of urinary excretion of calcium (to Control) |
|---|---|---|---|
| Control | - | 0.71 | - |
| Compound of Example 3 | 10 | 0.66 | 93% |
| Compound of Example 8 | 10 | 0.89 | 125% |
| Maxacalcitol | 9.4 | 1.5 | 211% |

As shown in Table 3, urinary excretion of calcium was nearly doubled in a group to which maxacalcitol was administered while, in the groups to which the compounds of the present invention were administered, there was nearly no effect on urinary excretion of calcium.

### Test Example 5: Investigation of metabolic rate in human liver microsome

Metabolic rates of the compounds of the present invention mentioned in Examples and maxacalcitol in human liver microsome were compared and investigated.
As to the pooled human liver microsome (HHM-0323), those manufactured by Tissue Transformation Technologies were used. A 0.25M potassium phosphate buffer (pH 7.4) (200 µL), 5 µL of a 250 µM solution of each compound to be tested in DMSO, 50 µL of an NADPH production system (where 20 mg of β-NADP⁺, 70 mg of glucose-6-phosphate, 40 units of glucose-6-phosphate dehydrogenase and 20 mg of magnesium chloride were dissolved in 1 mL of distilled water) and 220 µL of distilled water were added to a microtube and the mixture was subjected to a preincubation for 5 minutes at 37°C. Reaction was started by addition of 25 µL of human liver microsome (final concentration of protein: 1 mg/mL). Incubation was performed for 2, 10, 30 or 60 minutes and the reaction was stopped by addition of 500 µL of acetonitrile. After centrifugation at 13,000 rpm for 5 minutes, each test compound in the supernatant (25 µL) was measured by an HPLC under the following conditions.
Inertsil ODS-3 (4.6 x 150 mm; GL Science) was used as a column. With regard to mobile phase, a solution A (acetonitrile/0.1% aqueous solution of ammonium acetate = 10/90) and a solution B (acetonitrile/0.1% aqueous solution of ammonium acetate = 90/10) were used under the linear gradient conditions mentioned in Table 4. Column temperature, flow rate and wavelength for detection were set at 40°C, 1.0 mL/min. and UV 270 nm, respectively.

**Table 4**

| Time (minute(s)) | Solution A (%) | Solution B (%) |
|---|---|---|
| 0 | 55 | 45 |
| 10 | 10 | 90 |
| 10.01 | 55 | 45 |
| 16 | Measurement finished | |

From the result of measurement by the above-mentioned HPLC, metabolic rates of the test compounds were calculated. The result is shown in Table 5.

**Table 5**

| Tested Compound | Metabolic Rate (pmol/min/mg) |
|---|---|
| Maxacalcitol | 10 |
| Compound of Example 1 | 78 |
| Compound of Example 3 | 22 |
| Compound of Example 8 | 38 |
| Compound of Example 10 | 58 |
| Compound of Example 11 | 23 |
| Compound of Example 13 | 38 |
| Compound of Example 20 | 39 |

As shown in Table 5, the compounds of the present invention include compounds which are susceptible to metabolism in human liver microsomes as compared to maxacalcitol.

### Formulation Example 1:

Ointment (1 g) is prepared from 0.25 µg of the compound of Example 1, an ointment base (white Vaseline, medium-chain fatty acid triglyceride, lanoline, paraffin or a mixed base thereof) and other appropriate additives by means of kneading, etc. according to a conventional method.

### Formulation Example 2:

Ointment (1g) is prepared from 0.25 µg of the compound of Example 2, an ointment base (white Vaseline, medium-chain fatty acid triglyceride, lanoline, paraffin or a mixed base thereof) and other appropriate additives by means of kneading, etc. according to a conventional method.

### [Industrial Applicability]

As mentioned above, the compound of the present invention is a novel and useful vitamin D₃ derivative which do not have much effect on the systemic calcium metabolism while retaining an excellent vitamin D₃ activity.

## Claims

1. A 9,10-secopregnane derivative represented by the following general formula [1] or a pharmaceutically acceptable salt thereof. 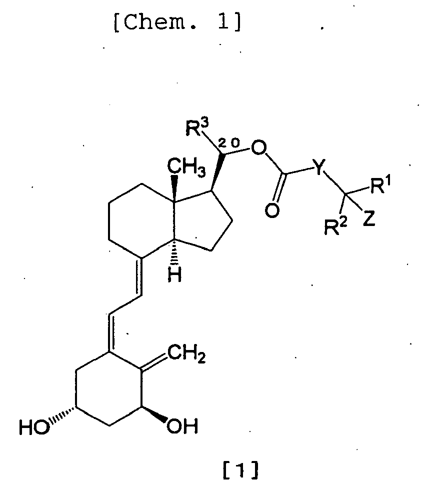 In the general formula [1],
Y represents (1) a single bond, (2) a C₁₋₅ alkylene which may be substituted by one to three member(s) selected from the group consisting of halogen, hydroxy and oxo, (3) a C₂₋₅ alkenylene or (4) phenylene;
R¹ and R² are same or different and each represents (1) hydrogen, (2) a C₁₋₆ alkyl which may be substituted by one to three halogen (s) or (3) a C₃₋₈ cycloalkyl, R¹ and R² taken together with the carbon atom adjacent thereto may form a C₃₋₈ cycloalkyl;
R³ represents hydrogen or methyl; and
Z represents (1) hydrogen, (2) hydroxy or (3) - NR¹¹R¹² in which R¹¹ represents hydrogen or a C₁₋₆ alkyl and R¹² represents (1) a C₁₋₆ alkyl which may be substituted by hydroxy or (2) a C₁₋₆ alkylsulfonyl.

2. The 9,10-secopregnane derivative according to claim 1, wherein Z is hydroxyl or a pharmaceutically acceptable salt thereof.

3. The 9,10-secopregriane derivative according to claim 1, wherein Y is a C₁₋₅ alkylene or a pharmaceutically acceptable salt thereof.

4. The 9,10-secopregnane derivative according to claim 1, wherein R¹ and R² are same or different and each is a C₁₋₆ alkyl or a pharmaceutically acceptable salt thereof.

5. The 9,10-secopregnane derivative according to claim 1, wherein R³ is methyl or a pharmaceutically acceptable salt thereof.

6. The 9,10-secopregnane derivative according to claim 1, wherein (1) Z is hydroxy, (2) Y is methylene or ethylene, (3) R¹ and R² are same or different and each is methyl or ethyl and (4) R³ is methyl or a pharmaceutically acceptable salt thereof.

7. The 9,10-secopregnane derivative according to claim 1, which is selected from the group consisting of the following compounds (1) to (33) or a pharmaceutically acceptable salt thereof.
(1) (1S,3R,20S)-20-(4-hydroxy-4-methylpentanoyloxy)-9, secopregna-5Z, 7E, 10 (19)-trien-1,3-diol,
(2) (1S, 3R, 20S) -20- (3-hydroxy-3-methylbutanoyloxy) -9, 10-secopregna-5Z,7E,10(19)-trien-1,3-diol,
(3) (1S, 3R, 20S) -20- (5-hydroxy-5-methylhexanoyloxy) -9, 10-secopregna-5Z,7E,10(19)-trien-1,3-diol,
(4) (1S,3R,20S)-20-(4,4,4-trifluoro-3-hydroxy-3-methylbutanoyloxy)-9,10-secopregna-5Z, 7E, 10 (19)-trien-1,3-diol,
(5) (1S,3R,20S)-20-(3-hydroxy-4-methyl-pentanoyloxy)-9,10-secopregna-5Z,7E,10(19)-trien-1,3-diol,
(6) (1S,3R,20S)-20-(4,4,4-trifluorobutanoyloxy)-9,10-secopregna-5Z,7E,10(19)-trien-1,3-diol,
(7) (1S,3R,20S)-20-[4,4,4-trifluoro-3-hydroxy-3-(trifluoromethyl)butanoyloxy]-9,10-secopregna-5Z,7E,10(19)- trien-1, 3-diol,
(8) (1S,3R,20S)-20-[(2E)-4-hydroxy-4-methylpent-2-enoyloxy]-9,10-secopregna-5Z,7E,10 (19)-trien-1,3-diol,
(9) (1S, 3R, 20S) -20- (3-cyclopropyl-3-hydroxypropanoyloxy)-9,10-secopregna-5Z,7E,10(19)-trien-1,3-diol,
(10) (1S,3R,20S)-20-[(2E)-4-ethyl-4-hydroxyhex-2-enoyloxy]-9,10-secopregna-5Z,7E,10(19)-trien-1,3-diol,
(11) (1S,3R;20S)-20-(5-hydroxy-5-methylheptanoyloxy)-9,10-secopregna-5Z, 7E, 10 (19)-trien-1,3-diol,
(12) (1S;3R,20S)-20-(3-ethyl-3-hydroxypentanoyloxy)-9,10-secopregna-SZ,7E,10(19)-trien-1,3-diol,
(13) (1S,3R,20S)-20-(4-ethyl-4-hydroxyhexanoyloxy)-9,10-secopregna-5Z,7E,10(19)-trien-1,3-diol,
(14) (1S,3R,20S)-20-[3-(1-hydroxy-1-methylethyl)-benzoyloxy]-9,10-secopregna-5Z,7E,10(19)-trien-1,3-diol,
(15) (1S,3R,20S)-20-[N-(isopropylsulfonyl)-3-aminopropanoyloxy]-9,10-secopregna-5Z,7E,10(19)-trien-1,3-diol,
(16) (1S,3R,20S)-20-(6-hydroxy-6-methylheptanoyloxy)-9,10-secopregna-5Z,7E,10(19)-trien-1,3-diol,
(17) (1S,3R,20S)-20-{4-[2,2,2-trifluoro-1-hydroxy-1-(trifluoromethyl)ethyl]benzoyloxy}-9,10-secopregna-5Z,7E,10(19)-trien-1,3-diol,
(18) (1S,3R,20S)-20-(5,5,5-trifluoropentanoyloxy)-9,10-secopregna-5Z,7E,10(19)-trien-1,3-diol,
(19) (1S,3R,20S)-20-[N-(2-hydroxy-2-methylpropyl)-N-methyl-2-aminoacetyloxy]-9,10-secopregna-5Z,7E,10(19)-trien-1,3-diol,
(20) (1S,3R,20S)-20-[3-(1-hydroxycyclopentyl)-propanoyloxyl-9,10-secopregna-5Z,7E,10(19)-trien-1,3-diol,
(21) (1S,3R,20S)-20-(3,3-difluoro-4-hydroxy-4-methylpentanoyloxy)-9,10-secopregna-5Z,7E,10(19)-trien-1,3-diol,
(22)(1S, 3R, 20S) -20- (3S)-3,4-dihydroxy-4-methylpentanoyloxy]-9,10-secopregna-5Z,7E,10(19)-trien-1, 3-diol,
(23) (1S,3R,20S)-20-(5,5,5-trifluoro-4-hydroxy-4-methylpentanoyloxy)-9,10-secopregna-5Z,7E,10(19)-trien-1, 3-diol,
(24) (1S,3R,20R)-20-(4-hydroxy-4-methylpentanoyloxy)-9,10-secopregna-5Z,7E,10(19)-trien-1,3-diol,
(25) (1S,3R,20 R)-20-(3-hydroxy-3-methylbutanoyloxy)-9,10-secopregna-5Z,7E,10(19)-trien-1,3-diol,
(26) (1S, 3R, 17β)-17-(4-hydroxy-4-methylpentanoyloxymethyl)-9,10-secoandrosta-5Z,7E,10(19)-trien-1,3-diol,
(27) (1S,3R,20S)-20-(4-hydroxy-4-methyl-3-oxopentanoyloxy)-9,10-secopregna-5Z,7E,10(19)-trien-1,3-diol,
(28) (1S,3R,20S)-20-(4-ethyl-4-hydroxy-3-oxohexanoyloxy)-9,10-secopregna-5Z,7E,10(19)-trien-1,3-diol,
(29) (1S; 3R, 20S)-20-[3-(hydroxymethyl)phenylacetyloxy]-9,10-secopregna-5Z,7E,10(19)-trien-1,3-diol,
(30) (1S, 3R, 17β)-17- [(2E)-4-ethyl-4-hydroxyhex-2-enoyloxymethyl]-9,10-secoandrosta-5Z,7E,10(19)-trien-1,3-diol,
(31) (1S,3R,20S)-20-[(3R)-3,4-dihydroxy-4-methylpentanoyloxy]-9,10-secopregna-5Z,7E,10(19)-trien-1,3-diol,
(32) (1S,3R,20S)-20-[5,5,5-trifluoro-4-hydroxy-4-(trifluoromethyl)pentanoyloxy]-9,10-secopregna-5Z,7E,10(19)-trien-1,3-diol, and
(33) (1S,3R,20S)-20-(3-hydroxy-3-n-propylhexanoyloxy)-9,10-secopregna-5Z, 7E, 10 (19)-trien-1,3-diol.

8. A pharmaceutical composition comprising the 9,10-secopregnane derivative according to any one of claims 1 to 7 or a pharmaceutically acceptable salt thereof as an active ingredient.

9. A treating agent for keratotic disorders comprising the 9,10-secopregnane derivative according to any one of claims 1 to 7 or a pharmaceutically acceptable salt thereof as an active ingredient.

10. A treating agent for psoriasis vulgaris comprising the 9,10-secopregnane derivative according to any one of claims 1 to 7 or a pharmaceutically acceptable salt thereof as an active ingredient.

11. A process for producing the 9,10-secopregnane derivative according to claim 1 or a pharmaceutically acceptable salt thereof, comprising a step of using a mixed anhydride represented by the following general formula [3a]. 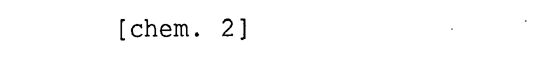 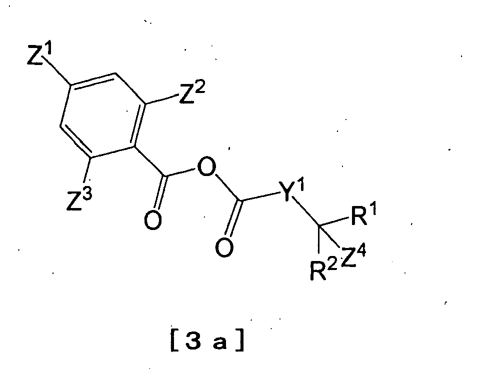 In the general formula [3a],
Y¹ represents (1) a single bond, (2) a C₁₋₅ alkylene which may be substituted by 1 to 3 member(s) selected from the group consisting of halogen, protected hydroxy and oxo, (3) a C₂₋₅ alkenylene or (4) phenylene;
R¹ and R² are same or different and each represents (1) hydrogen, (2) a C₁₋₆ alkyl which may be substituted by one to three halogen (s) or (3) a C₃₋₈ cycloalkyl, or R¹ and R² taken together with the carbon atom adjacent thereto may form a C₃₋₈ cycloalkyl;
Z¹, Z² and Z³ are same or different and each represents halogen; and
Z⁴ is (1) hydrogen, (2) protected hydroxy or (3) - NR¹³R¹⁴ where R¹³ represents hydrogen or a C₁₋₆ alkyl and R¹⁴ represents (1) a C₁₋₆ alkyl which may be substituted by protected hydroxy or (2) a C₁₋₆ alkylsulfonyl.

12. The process for producing the 9,10-secopregnane derivative according to claim 11 or a pharmaceutically acceptable salt thereof, wherein all of Z¹, Z² and Z³ are chlorines.

13. The process for producing the 9,10-secopregnane derivative according to claim 11 or a pharmaceutically acceptable salt thereof, wherein Y¹ is a C₁₋₅ alkylene.

14. The process for producing the 9,10-secopregnane derivative according to claim 11 or a pharmaceutically acceptable salt thereof, wherein R¹ and R² are same or different and each is a C₁₋₆ alkyl.

15. The process for producing the 9,10-secopregnane derivative according to claim 11 or a pharmaceutically acceptable salt thereof, wherein Z⁴ is a protected hydroxy and the protective group for hydroxy is trialkylsilyl, benzyl, (2-trimethylsilyl)ethoxymethyl, acyl or 2-tetrahydropyranyl.

16. The process for producing the 9,10-secopregnane derivative according to claim 11 or a pharmaceutically acceptable salt thereof, wherein (1) all of Z¹, Z² and Z³ are chlorines, (2) Y¹ is methylene or ethylene, (3) R¹ and R² are same or different and each is methyl or ethyl and (4) Z⁴ is a protected hydroxy and the protective group for hydroxy is trialkylsilyl, benzyl, (2-trimethylsilyl)ethoxymethyl, acyl or 2-tetrahydropyranyl.

17. A process for producing the 9,10-secopregnane derivative according to claim 1 or a pharmaceutically acceptable salt thereof comprising the following steps.
(a) a step for producing a compound represented by the general formula [4] by the reaction of a compound represented by the general formula [2] with a mixed anhydride represented by the general formula [3a]; and
(b) a step for producing a compound represented by the general formula [1] by deprotecting the protective group of each hydroxy in the compound represented by the general formula [4]. 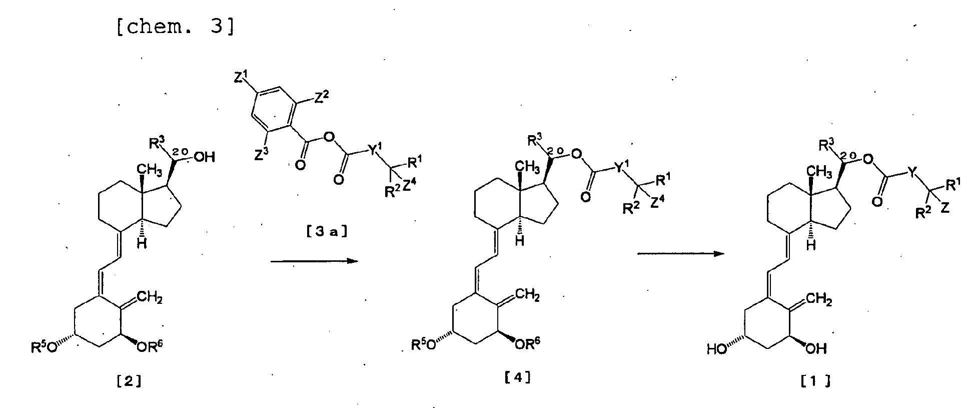 In the formulae,
Y represents (1) a single bond, (2) a C₁₋₅ alkylene which may be substituted by one to three member(s) selected from the group consisting of halogen, hydroxy'and oxo, (3) a C₂₋₅ alkenylene or (4) phenylene;
Y¹ represents (1) a single bond, (2) a C₁₋₅ alkylene which may be substituted by one to three member(s) selected from the group consisting of halogen, protected hydroxy and oxo, (3) a C₂₋₅ alkenylene or (4) phenylene;
R¹ and R² are same or different and each represents (1) hydrogen, (2) a C₁₋₆ alkyl which may be substituted by one to six halogen(s) or (3) a C₃₋₈ cycloalkyl, or R¹ and R² taken together with the carbon atom adjacent thereto may form a C₃₋₈ cycloalkyl;
R³ represents hydrogen or methyl;
R⁵ and R⁶ are same or different and each represents a protective group for hydroxy;
Z represents (1) hydrogen, (2) hydroxy or (3) - NR¹¹R¹² in which R¹¹ represents hydrogen or a C₁₋₆ alkyl and R¹² represents (1) a C₁₋₆ alkyl which may be substituted by hydroxy or (2) a C₁₋₆ alkylsulfonyl;
Z¹, Z² and Z³ are same or different and each represents halogen; and
Z⁴ represents (1) hydrogen, (2) protected hydroxy or (3) -NR¹¹R¹² where R¹¹ represents hydrogen or a C₁₋₆ alkyl and R¹² represents (1) a C₁₋₆ alkyl or (2) a C₁₋₆ alkylsulfonyl which may be substituted by protected hydroxy.

18. The process for producing the 9,10-secopregnane derivative according to claim 17 or a pharmaceutically acceptable salt thereof, wherein all of Z¹, Z² and Z³ are chlorines.

19. The process for producing the 9,10-secopregnane derivative according to claim 17 or a pharmaceutically acceptable salt thereof, wherein Z⁴ is a protected hydroxy and the protective group for hydroxy is trialkylsilyl, benzyl, (2-trimethylsilyl)ethoxymethyl, acyl or 2-tetrahydropyranyl.

20. The process for producing the 9,10-secopregnane derivative according to claim 17 or a pharmaceutically acceptable salt thereof, wherein Y and Y¹ each is methylene or ethylene.

21. The process for producing the 9, 10-secopregnane derivative according to claim 17 or a pharmaceutically acceptable salt thereof, wherein R¹ and R² are same or different and each is methyl or ethyl.

22. The process for producing the 9,10-secopregnane derivative according to claim 17 or a pharmaceutically acceptable salt thereof, wherein R³ is methyl.

23. The' process for producing the 9,10-secopregnane derivative according to claim 17 or a pharmaceutically acceptable salt thereof, wherein R⁵ and R⁶ are same or different and each is trialkylsilyl, benzyl, (2-trimethylsilyl)ethoxymethyl, acyl or 2-tetrahydropyranyl.

24. The process for producing the 9,10-secopregnane derivative according to claim 17 or a pharmaceutically acceptable salt thereof, wherein (1) all of Z¹, Z² and Z³ are chlorines, (2) Z⁴ is a protected hydroxy and the protective group for hydroxy is trialkylsilyl, benzyl, (2-trimethylsilyl)ethoxymethyl, acyl or 2-tetrahydropyranyl, (3) Y and Y¹ each is methylene or ethylene, (4) R¹ and R² are same or different and each is methyl or ethyl, (5) R³ is methyl and (6) R⁴, R⁵ and R⁶ are same or different and each is trialkylsilyl, benzyl, (2-trimethylsilyl)ethoxymethyl, acyl or 2-tetrahydropyranyl.
